# EUROPEAN PATENT SPECIFICATION

(11) **EP 3 654 893 B1**
(45) Date of publication and mention of the grant of the patent: **21.09.2022**
(21) Application number: 18835138.1
(22) Date of filing: 17.07.2018
(51) Int. Cl.: A61F 5/56, A61N 1/36, A61B 5/0205, A61B 5/00

(54) **A SYSTEM FOR AIDING EARLY DETECTION AND MANAGEMENT OF BREATHING RELATED DISORDERS**
SYSTEM ZUR UNTERSTÜTZUNG DER FRÜHERKENNUNG UND VERWALTUNG VON ATMUNGSBEDINGTEN STÖRUNGEN
SYSTÈME D'AIDE À LA DÉTECTION ET À LA PRISE EN CHARGE PRÉCOCES DE TROUBLES ASSOCIÉS À LA RESPIRATION

(30) Priority: 17.07.2017 AU 2017902791
(43) Date of publication of application: 27.05.2020
(73) Proprietor: Health Apps Pty Ltd, Kew, Victoria 3101 (AU)
(72) Inventor: GIANNOUKOS, John, Templestowe, Victoria 3106 (AU); KORFIATIS, Arthur, Balwyn North, Victoria 3104 (AU)
(74) Representative: Rigler, Johann
(86) International application number: PCT/AU2018/050751
(87) International publication number: WO 2019/014715

(56) References cited:
- WO-A1-2007/140584
- WO-A1-2016/170005
- WO-A2-02/13677
- US-A- 5 123 425
- US-A1- 2008 021 506
- US-A1- 2008 195 173
- US-A1- 2008 308 112
- US-A1- 2011 295 083
- US-A1- 2014 180 036
- US-A1- 2014 228 711
- US-A1- 2014 371 547
- US-A1- 2015 018 895
- US-A1- 2015 173 672

## Description

### Field of the Invention

The invention relates to a system for aiding early detection and management of breathing related disorders and in particular to a system for aiding management of disorders such as sleep apnea (apnea) or snoring or sudden infant death syndrome (SIDS) or infant apnea.

The invention has been developed primarily for use in/with health industry and self- help health industry for aiding assessing and/or pre-treating of sleep apnea and will be described hereinafter with reference to this application.

It will be appreciated that the invention is not limited to this particular field of use.

### Background of the Invention

A system for aiding early detection and management of breathing related disorders is usually a treatment that is implemented after the event of the disorder has fully developed. This is the case when managing snoring or sleep apnea. In other breathing related disorders, it is possible that there is no pre-warning or normal symptoms and therefore often fatalities occur.

Millions of people around the world are dependent on continuous positive airway pressure (CPAP) machines to help them manage snoring and sleep apnea. CPAP devices deliver a stream of compressed air via a hose and mask, attempting to keeping the airway open under air pressure, and enabling improved / unobstructed breathing.

CPAP machines are cumbersome, bulky, noisy and can in themselves be the cause of awakening people with Sleep Apnea. The success of a CPAP machine is reliant on correct fitting, avoiding airleaks and continued use of cumbersome fitout of tubes. They can also be detrimental or aid to complications in people with heart disease or who have suffered a stroke. In some people CPAP machines prevent restful sleep and their use is associated with high blood pressure, arrhythmia, stroke and heart failure. Heart disease is the leading cause of death in the United States, and stroke is also a leading cause of death and disability. It therefore cannot be considered to be the solution for all cases and alternatives are needed. A large percentage of people stop using CPAP machines in first few months.

The trouble with these problems is that millions of people around the world are dependent on continuous positive airway pressure (CPAP) machines to help them manage snoring and sleep apnea. Also, it is important in most medical fields to have preventative measures rather than treatment measures.

It can be seen that breathing related disorders have one or more of the problems of:
a) Minor breathing or non-breathing interruptions;
b) Breathing disorder effects such as snoring,
c) Breathing difficulties which is emphasized by startling reflexes, smothering or other direct physical effect;
d) Uncomfortableness while sleeping due to variable breathing resulting in heat and oxygen variation where not wanted
e) Indirect effects such as lack of oxygen circulation;
f) Dire consequences such as lack of oxygen to the brain; g) the inappropriateness of internal aids; or
h) risks associated with surgical procedures.

It is known to have sleep apnea treatment systems. One form is a CPAP machine. Some of the inconveniences of a CPAP machine include:
. It's intrusive and cumbersome to wear the mask.
   - Difficult to get a good night's sleep as the mask, air tubes, air leaks, pump noise continuously awakens you.
   - Monitoring and assessment is not easy or straight forward, usually requires medical personal to assist and to analyse any data the CPAP machine may collect.
. Once a CPAP machine has been recommended for users, there has been a missed opportunity to diagnose and manage at an earlier stage.

It is known to have systems which are implanted into the patient. These have the inconveniences of:
a) Expensive surgery; b) Very invasive;
c) Not readily changeable;
d) Expensive and therefore not available to most;
e) Only used in very critical cases due to expense and therefore not available for prevention or early diagnosis of critical cases;
f) Inability to upgrade hardware without additional surgery;
g) If implant solution does not offer expected results - additional surgery for removal.

US 2015/018895 A1 discloses an electric stimulation method and system for treating Obstructive Sleep Apnea (OSA) Syndrome using an external actuator on the pharyngeal-laryngeal muscles. Using this actuator, the muscles involved receive an electric stimulus, with the aim of widening the muscular opening and attaining sufficient air flow to prevent the lack of air. Said stimulus only acts in the event of an apnea episode being detected, by means of analyzing sound patterns of the upper airways. Both detection and treatment arc self-regulatory, in order to adapt to the patient's morphology and the evolution of the condition or problem.

US 2014/371547 A1 relates to sleep monitoring and stimulation comprises collecting actigraphy data from a user. The user's sleep phase is determined using the actigraphy data. At least one stimulation, determined at least in part on the sleep phase, is directed towards the user. Subsequent actigraphy data is collected from the user. The actigraphy data and subsequent actigraphy data is used to determine the user's subsequent sleep phase. The stimulation is modified, based at least in part upon the subsequent sleep phase.

US 2014/228711 A1 discloses a device for sleep apnea avoidance and data collection may include a sensor configured to sense a pressure and generate a first signal when the pressure exceeds a threshold. A signal generator module may be configured to generate a first stimulating signal in response to the first signal. The sensed pressure may include a pressure exerted on the sensor when a user of the device lies down on the device. The first stimulating signal may be configured to cause the user to change sleeping position, for example, from a first sleeping position that causes snoring to a second sleeping position that stops snoring.

US 2014/180036 A1 relates to a wireless sleep apnea treatment system comprises a garment having at least one ECG monitor, a wireless signal acquisition board in communication with the ECG monitor and the computer and providing the electrical reading from the ECG monitor to the computer, and a patient stimulator controlled by the computer through the wireless signal acquisition board.

US 2011/295083 A1 pertains to a therapeutic and diagnostic systems and methods help an individual with a sleep disordered breathing condition, such as habitual snoring or obstructive sleep apnea (OSA), achieve deep, restorative sleep. The systems and methods include component that serve complementary sensing, monitoring, and corrective or diagnostic functions.

US 2008/308112 A1 discloses a system and method for reducing snoring and sleep apnea of a sleeping person, the system comprising at least one sensor for detecting occurrence and/or likeliness of occurrence of the snoring and/or sleep apnea and for producing a sensor signal indicative of the occurrence and/or likeliness of the occurrence of the snoring and sleep apnea and a processor unit for determining from the sensor signal whether the occurrence of the snoring and/or sleep apnea of the sleeping person is likely, wherein the system is provided with a stimulator controllable by the processor unit, wherein the stimulator is arranged to trigger the sleeping person to change position using a triggering signal.

US 2008/021506 A1 relates to a device and method for the treatment of sleep apnea and/or snoring in a human patient includes at least one electrode stimulator for providing direct electrical stimulation to a throat and/or laryngeal muscles of the patient. The stimulator is constructed and arranged to be positioned at the throat and/or laryngeal muscles of the patient, either on the surface or subcutaneously. A power source provides a continuous electrical signal to the stimulator so that the throat and/or laryngeal muscles of the patient are contracted to open the airway of the patient. The power source can be a portable source remote from the electrode or a pacemaker unit also implanted in the patient.

WO 2007/140584 A1 presents a method and device for treating snoring and obstructive sleep apnea, in which one or more sensors sense the onset of an episode of snoring or sleep apnea and cause production of an electrical stimulation signal consisting of a train of alternating pulses. The pulse intensity is ramped up from a low level to a level high enough to end the episode. The end of the episode is sensed and the stimulation signal is then ramped down or turned off. Therefore, while the intensity of the stimulation signal has become high enough to end the undesired episode, the intensity does not become higher than that needed for this purpose. Therefore, the stimulation signal is less likely to disturb the sleep of the person being treated.

WO 2002/13677 A2 discloses a system and method for treating obstructive sleep apnea (OSA) is disclosed, whereby the apnea event is terminated prior to cessation of breathing. The system comprises at least one microphone, which generate breathing sound signals sent to a controller. The controller digitally identifies at least one signal as associated with the breating pattern of a user indicative of the onset of OSA. When the controller detects a signal indicative of OSA onset, an alarm signal is sent to a generator. The generator stimulates a user, causing the user to move in manner that terminates the OSA event before cessation of breating occurs. This OSA event termination method occurs without waking the user and simultaneously without causing physiological stress associated with cessation of breathing. US 5123425 A relates to a system for the treatment of obstructive sleep apnea packaged in a collar which can be worn by a patient without any special preparation.

It can be seen that known techniques for treating breathing related disorders have a range of problems and the present invention seeks to provide a system for aiding management of breathing related disorders, which will overcome or substantially ameliorate at least one or more of the deficiencies of the prior art, or to at least provide an alternative.

It is to be understood that, if any prior art information is referred to herein, such reference does not constitute an admission that the information forms part of the common general knowledge in the art, in Australia or any other country.

### Summary of the Invention

The invention is defined in the appended claims. Aspects, embodiments and examples disclosed herein which do not fall within the scope of the appended claims do not form part of the invention, and are merely provided for illustrative purposes.

One aspect of the invention relates system for aiding early detection and management of a breathing related disorder in a patient comprising: a) a user controller: b) a plurality of sensors, comprising: an oximetry/O2 level sensor and at least one additional sensor, wherein the sensors are connectable to the user controller and adapted for sensing and providing sensed data of at least one characteristic relevant to a breathing related disorder; c) a predefined operative framework in the user controller having connection for receiving the sensed data from the plurality of sensors; d) a control means for sending to the predefined operative framework a predefined effective operative range of the plurality of sensors; e) a collaborator; and f) at least one external stimulator positionable relative to the patient and operatively connectable with the predefined operative framework for receiving operative instruction and providing an effective external stimulation to the patient; where i) the sensed data is assessed to determine whether a predefined trigger point has been sensed; and ii) If a predefined trigger point has been sensed an identification of the single sensor trigger point is communicated to the collaborator; and iii) the collaborator undertakes a trigger point analysis by reviewing multiple sensors, whereby: if multiple single sensor trigger points from multiple sensors have been received within a predefined time period a trigger actuation is initiated to instigate external stimulation; or if multiple single sensor trigger points from multiple sensors have not been received within a predefined time period return to further sensing and further trigger analysis; wherein the recordal of a single sensor trigger point of one sensor does not initiate trigger actuation unless supported by a single sensor trigger point of another sensor.

A further aspect of the invention relates to a system for aiding management of a breathing related disorder comprising: a) at least one sensor for sensing at least one characteristic of a breathing related disorder; b) an access port for upload of details of the at least one characteristic of a breathing related disorder from the sensor to an online computerised means; c) a receiver port; for download from the online computerised means, which is adapted for receiving computerised instructions according to a predefined effective treatment range in managing control of a breathing related disorder; d) at least one topical stimulator positionable on a user for providing an effective stimulation option to the user and including a receiver for receiving electronic control instructions; e) at least one input device for allowing a user to predefine at least one user particular stimulation output and provide for upload to the access port; f) at least one display device for allowing display of an external stimulation output to the user to provide a confirmed user defined management control of the breathing related disorder application; g) at least one transmitter for transmitting each user particular input of the plurality of users to the access port of the online means for upload to the online computerised means; and h) at least one receiver for transmitting for receiving by at least one of the at least one display device for allowing display of the respective confirmed user defined breathing related disorder application of each user; wherein the plurality of user particular inputs defines the predefined effective treatment range and control of a breathing related disorder and the selection of an effective stimulation option defines the chosen effective option and is provided to the at least one display device.

A still further aspect of the invention relates a method of automatically creating and running a freeform breathing related disorder using a computerised system including the steps of: a) providing a predefined operative framework identifying a plurality of stimulation options for selection by the user in their respective user particular input wherein a selection of an effective stimulation option from each category matching a user particular input of the same stimulation options in each category defines the chosen effective option; b) receiving the user defined breathing related disorder application from a user over a digital communication system connected to an access port for upload to an online computerised means adapted for following computerised instructions according to a predefined effective treatment range and control of a breathing related disorder; c) selecting an effective stimulation option according to the predefined operative framework; d) comparing the plurality of effective stimulation options from each category to each of confirmed user defined breathing related disorder application wherein a selection of an effective stimulation option from each category matching a user particular input of the same stimulation options in each category defines the chosen effective option.

Any disclosure herein which creates ambiguities in relation to the three aspects of the invention presented above does not form part of the invention.

The breathing related disorders can include one or more of:
- Sleep apnea • Snoring
- SIDS (Sudden Infant Death Syndrome)

Preferably the effective external stimulation by the at least one external stimulator effects an alteration to the user's breathing.

The effective external stimulation by the at least one external stimulator can be by indirectly effecting an alteration to the user's breathing. Such indirectly effecting of an alteration to the user's breathing can be by notification to the user.

The system for aiding early detection and management of breathing related disorders can have the effective external stimulation by the at least one external stimulator substantially directly effecting an alteration to the user's breathing. Such directly effecting of an alteration to the user's breathing can be selected from one or more of:
- an external stimulator locatable on or near the ear of the user providing acoustic output for stimulation to the user,
- an external stimulator locatable under chin area for stimulation to the user,
- an external stimulator locatable on the head for external stimulation to the user,
- an external stimulator locatable on the finger or limbs for stimulation to the user,
- an external stimulator using TENS (Transcutaneous Electrical Nerve Stimulation) or EMS (Electrical Muscle Stimulation) or both locatable to provide for stimulation to the user, or
- an external stimulator locatable on the foot for stimulation to the user.
- wherein the external stimulators can operate in a mode that directly affects a user to improve the breathing of the user.

Most preferably the communication between sensors, control and at least one 20 external stimulator is wired and/or wireless.

According to an aspect of the present invention, a system for aiding early detection and management of breathing related disorders is provided by an external stimulation of the tongue in order to improve openness of the airway.

It can be seen that the invention provides a system for the benefit of allowing ready non-invasive preventative, early management, and management tool for aiding management of breathing related disorders.

The invention in one particularly advantageous form has a system for aiding early detection and management of breathing related disorders including a plurality of the at least one external sensor and a control means for receiving to the predefined operative framework a predefined effective operative range of each of the plurality of the at least one external sensor; wherein sensing by at least two of the plurality of the at least one external sensor within the predefined effective treatment range and control aids early detection and management of a breathing related disorder by an effective sensing by the at least one sensor.

The control means can include a collaborator and each sensor receives sensed data and assesses if a predefined trigger point has been sensed and sends identification of single sensor trigger point to the collaborator so it can receive and undertake a trigger analysis including reviewing multiple sensors. If multiple single sensor trigger points from multiple sensors have been received within a predefined time period there is created a trigger actuation and outputting trigger output to operative framework to instigate external stimulation. If multiple single sensor trigger points from multiple sensors have not been received within a predefined time period there is a return to further sensing and further trigger analysis.

In this way the recordal of a single sensor trigger point of one sensor does not initiate trigger actuation unless supported by a single sensor trigger point of another sensor.

In a particular preferred form of multiple sensors the system for aiding early detection and management of breathing related disorders has the user controller including connection with one or more input selectable sensor modules providing the at least one sensor. The one or more input selectable modules includes a selected sensor internal module part for connection with the user controller and an external sensor module part for location on an external body location and for providing sensed data to the selected sensor internal module part.

Also in a preferred form there is provided a system for aiding early detection and management of breathing related disorders having the user controller including connection with one or more output selectable stimulator modules providing the at least one external stimulator. The one or more output selectable stimulator modules includes a selected stimulator internal module part for connection with the user controller and an external stimulator module part for location on an external body location and for providing stimulation to the selected stimulator external module part.

It can be seen that the invention of the system for aiding early detection and management of breathing related disorders provides the benefit of ready selection and connection of different sensors and different stimulators so that a particularly suitable and effective and acceptable system and device is provided for that particular user as per the user's choice, the medical practitioner's advice on breathing and sleeping disorders, the carer's choice or a combination thereof.

According to another aspect of the present invention, a system for aiding early detection and management of breathing related disorders is provided by there being provided an external stimulation of the hypoglossal nerve region and surrounding area around the submental tongue area in order to improve openness of the airway.

It can be seen that the invention of the system for aiding early detection and management of breathing related disorders provides the benefit of research which showed that the best way to stimulate the tongue is to stimulate the hypoglossal nerve/region, around the submental area. This in turn causes the tongue to contract based on level of stimulation set by user.

According to a further aspect of the present invention, a system for aiding early detection and management of breathing related disorders is provided by a variable external stimulation of the tongue in order to retain effectiveness of improving openness of the airway.

It can be seen that the invention of the system for aiding early detection and management of breathing related disorders provides the benefit of research which showed that the best way to continue to stimulate the tongue is to change the stimulation of the hypoglossal nerve/region, around the submental area. In this way the nerve does not become immune or numbed to the stimulation but is kept reactive and readily useable as an effective stimulant to the tongue to cause contraction and open the airway.

In one form the invention provides a system a system for aiding management of a breathing related disorder comprising at least one sensor for sensing a characteristic of a breathing related disorder, an access port for upload from the sensor to an online computerised means; a receiver port for download from the online computerised means, which is adapted for receiving computerised instructions according to a predefined effective treatment range in managing control of a breathing related disorder; at least one topical stimulator positionable on a user for providing an effective stimulation option to the user and including a receiver for receiving electronic control instructions; at least one input device for allowing a user to predefine at least one user particular stimulation output and provide for upload to the access port; at least one display device for allowing display of an external stimulation output to the user to provide a confirmed user defined management control of the breathing related disorder application; at least one transmitter for transmitting each user particular input of the plurality of users to the access port of the online means for upload and transfer of data with the online computerised means; and at least one receiver for transmitting for receiving by at least one of the at least one display device for allowing display of the respective confirmed user defined breathing related disorder application and functions of each user; wherein the plurality of user particular inputs defines the predefined effective treatment range and control of a breathing related disorder and the selection of an effective stimulation option defines the chosen effective option and is provided to the at least one display device; wherein the system for aiding early detection and management of breathing related disorders can be substantially assembled with improving effectiveness in aiding early detection and management of breathing related disorders including any one or more of the following:
i. improvements in structure and assembly including ease of operation in order to allow ready external use; ii. Improvements in stimulation including better operation for self-use; iii. Improvements in control of variation of stimulation by providing predefined options and selection techniques; iv. is easy to use by people and control the unit using their smart device such as computers, smart phones, health fitness wrist bands, tablets and watches, with interacting via cable or wireless including WiFi, Bluetooth or other wireless technologies. v. The Health App comes with endless opportunities to manage awareness, inform family, medical support people or any support or carer person for identifying opportunities to improve management plan, align these activities to other health manage plans to assess overall outcomes, etc and vi. The Health App updates will be easily applied onto the user's device or manually as needed by user.

It can be seen that the invention of a system for aiding early detection and management of breathing related disorders provides the benefit of non-invasive preventative and management tool of aiding management of disorders such as sleep apnea or snoring or sudden infant death syndrome (SIDS).

The invention can deliver a small portable solution that is simple to use, enables people to better assess and manage their apnea /snoring condition and delivers improved overall early management, significantly reducing the risk of related chronic disorders as people are more likely to continue using a more comfortable solution at an early stage and potentially delay moving to a CPAP machine is significantly cheaper than surgical options without associated risks.

The invention can provide multiple combination of external stimulations and notifications either concurrently, sequentially or as selected.

Further benefits of this system are that it is easy to use, does not require professional and/or medical support to use, is significantly cheaper than a CPAP machine and delays the transition to a CPAP machine.

### Other aspects of the invention are also disclosed. Brief Description of the Drawings

Notwithstanding any other forms which may fall within the scope of the present invention, preferred embodiments of the invention will now be described, by way of example only, with reference to the accompanying drawings in which:
Fig. 1 is a general diagrammatic view of a system for aiding management of a breathing related disorder for use in the health industry and self-help health industry in accordance with a preferred embodiment of the present invention;
Fig. 2 is a diagrammatic view of multiple sensors with primary sensors and secondary sensors for use in an embodiment of the system of Fig. 1 ; Figs. 3, 4 and 5 are diagrammatic views of user controller with choice of a selection of sensor modules and details of the internal sensor module part and external sensor module part;
Fig. 6 is a diagrammatic view of the selection of categories of location of sensors that can be for use in the system for aiding management of a breathing related disorder of Fig. 1 ;
Fig. 7 is a diagrammatic view of the selection of categories of mode of operation of sensors that can be for use in the system for aiding management of a breathing related disorder of Fig. 1 ;
Fig. 8 is an explanatory cutaway of a detail of the hypoglossal nerves that can be activated externally from the throat to improve breathing in system for aiding management of a breathing related disorder of Fig. 1 ;
Figs. 9, 10 and 11 are diagrammatic views of user controller with choice of a selection of external stimulator modules and details of the internal stimulator module part and external stimulator module part;
Fig. 12 is a diagrammatic view of the selection of categories of location of stimulators that can be for use in the system for aiding management of a breathing related disorder of Fig. 1 ;
Fig. 13 is a diagrammatic view of the selection of categories of mode of operation of stimulators that can be for use in the system for aiding management of a breathing related disorder of Fig. 1 ;
Fig. 14 is a diagrammatic block diagram of the operation of the sensors of the system for aiding management of a breathing related disorder of Fig. 1 ;
Fig. 15 is a diagrammatic block diagram of the operation of multiple of the sensors of the system for aiding management of a breathing related disorder of Fig. 1 ; Fig. 16 is a diagrammatic view of the timing operation of trigger actuation by multiple of the sensors of the system for aiding management of a breathing related disorder of Fig. 1 ;
Fig. 17 is a diagrammatic block diagram of the operation of the external stimulator of the system for aiding management of a breathing related disorder of Fig. 1 ; Fig. 18 is an overall diagrammatic view of a range of functional options of the system for aiding management of a breathing related disorder of Fig. 1 ; Fig. 19 is a diagrammatic view of the self-modifying operation of the sensors and external stimulators by feedback and control in a variable system for aiding management of a breathing related disorder of Fig. 1 ;
Fig. 20 is a diagrammatic view of the self-changing control of the sensor usable in the self-modifying operation of the sensors and external stimulators by feedback and control in a variable system for aiding management of a breathing related disorder of Fig. 1 ;
Fig. 21 is an operative flow diagram of the operation of the self-changing operation of the sensors of Fig. 20;
Fig. 22 is a diagrammatic view of the self-changing control of the external stimulator usable in the self-modifying operation of the sensors and external stimulators by feedback and control in a variable system for aiding management of a breathing related disorder of Fig. 1 ; and
Fig. 23 is an operative flow diagram of the operation of the self-changing operation of the external stimulators of Fig. 22.

### Description of Preferred Embodiments

It should be noted in the following description that like or the same reference numerals in different embodiments denote the same or similar features.

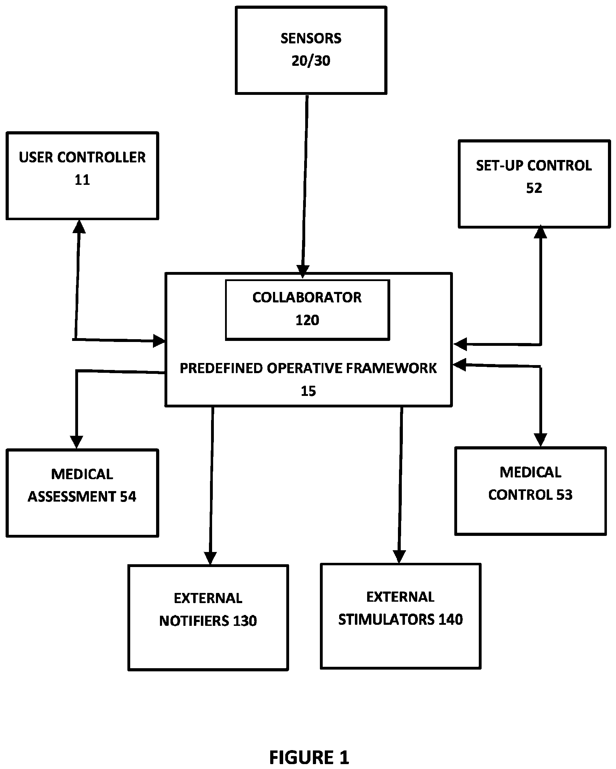

Referring to the drawings, in figure 1 there is shown a general application of a system for aiding early detection and management of a breathing related disorder in accordance with an embodiment of the invention. The system for a user has at least one sensor 20/30 from a plurality of sensors 20 for sensing at least one characteristic of a breathing related disorder. The breathing disorder can relate to breathing related disorders such as sleep apnea, snoring or even SIDS (Sudden Infant Death Syndrome).

There is provided a predefined operative framework 15 that is in receivable connection to the sensors 20 to receive information from the sensors about the sensed characteristic. The predefined operative framework 15 further has operable connection to at least one external stimulator of a plurality of stimulators 140 positionable relative to a user for providing an effective external stimulation option to the user.

The predefined operative framework 15 is connected or connectable to a range of other connections including the user controller for the user to be able to make input and assist control. In this regard the user controller 11 could be a separate device to the predefined operative controller 15 such as a mobile telephone connecting to a modem having the predefined operative controller 15. In another form the predefined operative controller 15 can be integral, insertable or downloadable into the user controller 1 1.

Apart from the predefined operative controller 15 being connectable to receive the user's input there can be a set-up control 52 that can be remote and able to be downloaded or integral or insertable into the user controller 11 or predefined operative controller 15. This allows for set-ups of sensors 20 and stimulators 140 according to their characteristics and according to set-up rules. The user controller 1 1 or predefined operative controller 15 is also connected or connectable to medical control 53 so that a medical practitioner can affect input controls according to medical practice, particularity of the particular user as a medical patient, or due to developments of medical and technical understanding of the operation of the sensors and stimulators in providing aids early detection and management of a breathing related disorder. An output of the medical assessment 54 can provide sensed data to the medical practitioner the user or other carers for use in aiding early detection and management of a breathing related disorder. There also can be simple external notifiers as triggers or advice of a detection by the device or system so that carers or those related to users are advised of sensed data or trigger points for use in aiding early detection and management of a breathing related disorder.

As will be further described the ability for selectable choice or change of sensors or change of operation of sensors and the selectable choice or change of stimulators or change of operation of stimulators is important in allowing this system to be adaptable to the most effective form for the user and to retain effectiveness as the user acclimatises or builds up a resistance to the single operation of use.

The system and device for aiding management of a breathing related disorder also acts as a gatherer of sensed data for use by medical practitioners for precise diagnosis rather than the self-use that is an aid to diagnosis, maintenance and treatment. Clearly though a user cannot be under 24-hour medical supervision seven days a week so a system to support and augment the relationship between user and medical practitioner is clearly advantageous. Also, the system and device provide a substantial minimization of oversight of indicators as this is dramatically reduced by the extended use of the aid by the user.

There is also a control means for providing to the at least one topical stimulator a predefined effective treatment range of the at least one topical stimulator positionable on the user in managing control of a breathing related disorder of the user. The control means can be to an external online computerised means or to an app on a user's smart telecommunication device or a combination.

The control means can provide a predefined effective treatment range of the one external stimulator positionable relative to a user, which control aids management of the breathing related disorder by an effective external stimulation or notification.

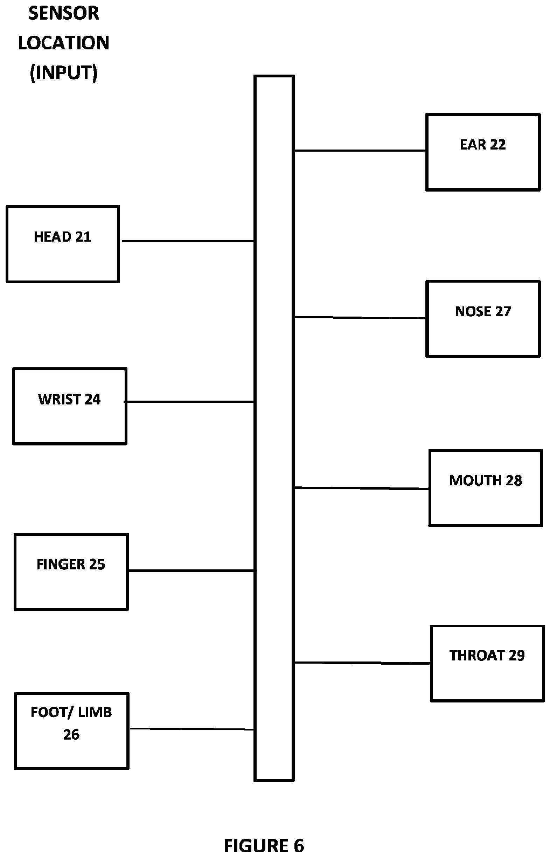

As shown in Fig. 6 the sensors 20 can be selected from one or more locations such as sensor 21 locatable on the head of the user, sensor 22 locatable near mouth or nose or lungs for detecting breath of user, sensor 23 locatable on the ear, sensor 24 locatable on the wrist, sensor 25 locatable on the finger or sensor 26 locatable on the foot and can be sensing characteristic that is relevant to the breathing of the user. The sensors 20 can operate in a mode that senses a particular characteristic that is relevant to the breathing of the user.

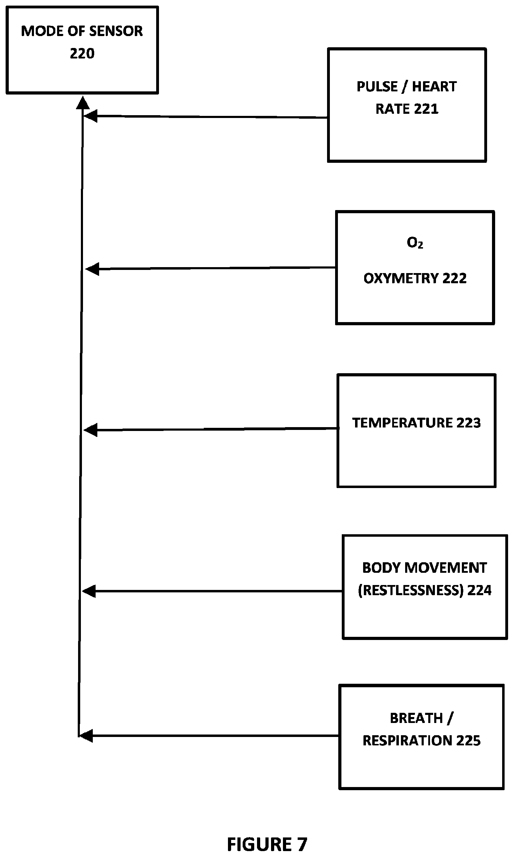

Referring to Fig. 7, the various modes 220 of sensor can be a mode 221 to detect the characteristic of the pulse/ heart rate, a mode 222 to detect the characteristic of oxygen levels, a mode 223 to detect the characteristic of temperature of the user, or a mode 224 to detect the characteristic of body movement or restlessness of the user and their settings. All of these modes 220 have a different operating range within which sensing will indicate a particular breathing effectiveness. Changes of the characteristic out of those ranges or sudden changes in that range can indicate prewarning issues of the breathing related disorders.

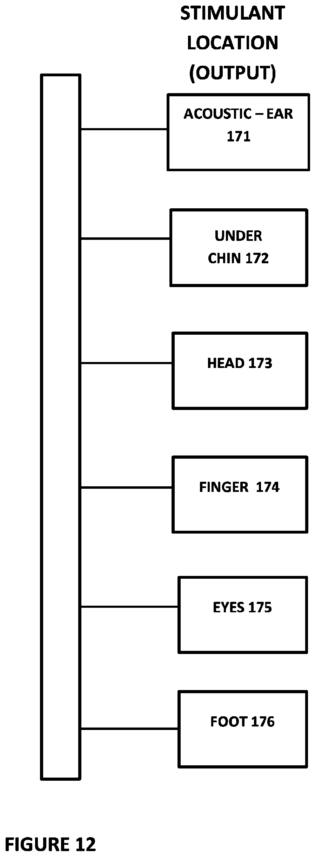

As shown in Fig. 12 the stimulators 140 can be selected from one or more locations such as external stimulator 41 locatable on or near the ear of the user providing acoustic output for stimulation or notification to the user, external stimulator 42 locatable under chin for stimulation or notification to the user, external stimulator 43 locatable on the head for external stimulation or notification to the user, external stimulator 44 locatable on the finger for stimulation or notification to the user, external stimulator 45 using TENS (Transcutaneous Electrical Nerve Stimulation) or EMS (Electrical Muscle Stimulation) or both locatable to provide for stimulation or notification to the user, or external stimulator 46 locatable on the foot for stimulation or notification to the user. The external stimulators 40 can operate in a mode that affects a user to improve the breathing of the user.

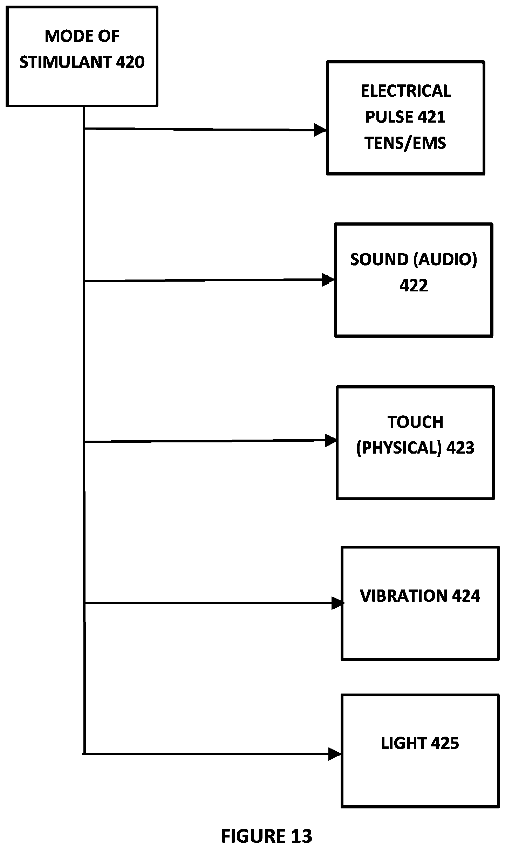

Referring to Fig. 13, the various modes 420 of external stimulators can be a mode 421 to provide electrical pulse or stimulation through TENS and/or EMS, a mode 422 to provide the external stimulation or notification by sound (audio), a mode 423 to provide the external stimulation or notification by touch (physical), or a mode 424 to provide the external stimulation or notification by vibration applied to the user. All of these modes 420 have a different operating range within which external stimulation or notification will improve a particular breathing effectiveness. Changes of the characteristic out of those ranges or sudden changes in that range can indicate prewarning issues of the breathing related disorders.

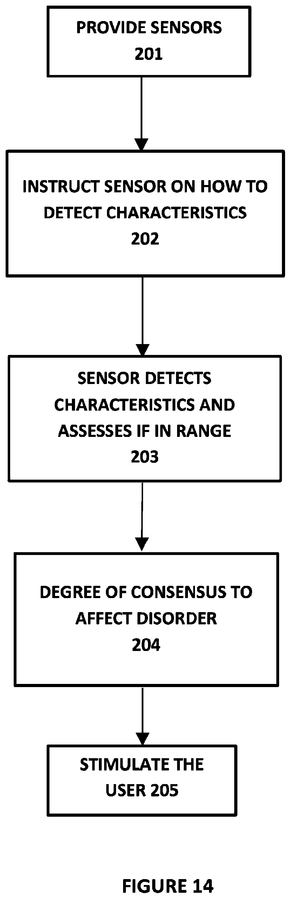

Referring to Fig. 14 there is shown a partial operation of the system of Fig. 1 in which in a first step 201 the selection of sensors 20 are provided. This allows a user to select one or more sensors 20 and allow connection to the predefined operative framework 15. Such framework can be provided on a smart device solely or in combination with a remote computer or cloud-based platform connected by wireless telecommunication.

In a second step 202 the predefined operative framework 15 which is operatively connected to the selected one or more of the sensors 20 and to the selected one or more of the external stimulators 140 and requires instructions on how to detect characteristics of the particular user using the particular sensor for the particular characteristics relevant to the breathing disorder. In effect, the sensors themselves do not need to be particularly pre-programmed and specialised for the purpose but can be smart devices that are set-up by the predefined operative framework 15. This can be achieved by such framework having predefined controls therein and/or by communication with a plurality of controls.

The controls can be a user control 51 such that a user can enter sensor product information and personal dimension information of relevance such as gender, weight, preconditions, condition of concern and other details. There also can be a medical control 53 that interprets the personal information and the expected breathing related disorders and provides a framework for the characteristic relevant to the breathing disorder. A third control can be a set-up control 52 that is determinative of the activating controls and sensitivities and calibration details of the selected sensor which allows for the specialised adaption in situ to the operation of the system.

As shown in Step 203 the sensor 20 detects the relevant characteristics relevant to the breathing disorder and assesses if in range as determined by the medical control 53. This can further include wireless communication to remote computer or cloud- based platform for such review or be maintained in a comparative review on the user's smart device.

In step 204 there is the review of the degree of consensus that would affect the disorder. In this regard, a false alarm is not beneficial and could stimulate when not required and cause stress to the user which exacerbates the situation rather than assists the situation. Therefore, the consensus can be to see the length of time that a sensor detects characteristics assessed in the relevant range or undertaking further sensing and if three sensed readings detected in the assessed range then consensus with the sensed characteristics does warrant action by the stimulator or notificator to the user in order to aid early detection and management of the breathing related disorder.

Thereby in Step 205 the external stimulator is activated according to the controls 51 , 52 and 53 within a predefined effective treatment range of the one external stimulator positionable relative to a user, and which aids management of the breathing related disorder by an effective external stimulation or notification.

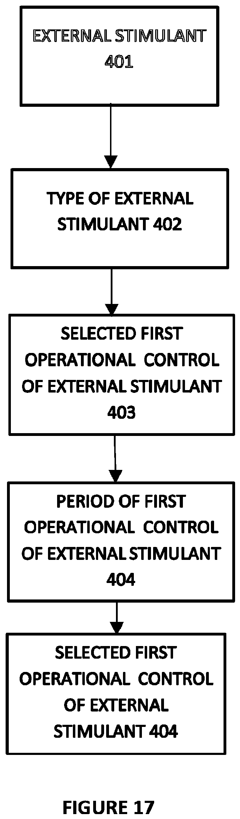

Referring to Fig. 17 there is shown a partial operation of the system of Fig. 1 in which in a first step 401 the selection of external stimulators 40 are provided. This allows a user to select one or more external stimulators 40 and allow connection to the predefined operative framework 15. Such framework can be provided on a smart device solely or in combination with a remote computer or cloud-based platform connected by wireless telecommunication.

In a second step 402 the predefined operative framework 15 which is operatively connected to the selected one or more of the external stimulators 40 and requires instructions on how to provide the effective stimulation or notification to the user to effect the response required to improve relevant breathing disorder. In effect, the external stimulators 40 themselves do not need to be particularly pre-programmed and specialised for the purpose but can be smart devices that are setup by the predefined operative framework 15. This can be achieved by such framework having predefined controls therein and/or by communication with a plurality of controls.

In step 403 there is the selected first operational control of the external stimulant by the controls can be the user control 51 such that a user can enter external stimulator product information and personal dimension information of relevance such as gender, weight, preconditions, condition of concern and other details. There also can be the medical control 53 that interprets the personal information and the expected breathing related disorders and provides a framework for the determined treatment stimulation or notification relevant to assess, pre-warn or improve the breathing disorder. A third control can be the set-up control 52 that is determinative of the activating controls and sensitivities and calibration details of the selected external stimulators which allows for the specialised adaption in situ to the operation of the system.

In step 404 there is the determination of the periodic time of stimulation and/or the length of operation of the external stimulator 140. This can be in reference to the user control 51 , set-up control 52 and medical control 53.

Then by Step 405 the at least one external stimulator 140 positionable relative to a user and operatively connectable with the predefined operative framework for provides an effective external stimulation to the user within a predefined effective operative range of the at least one external stimulator and wherein the predefined effective treatment range and control aids early detection and management of a breathing related disorder by an effective external stimulation.

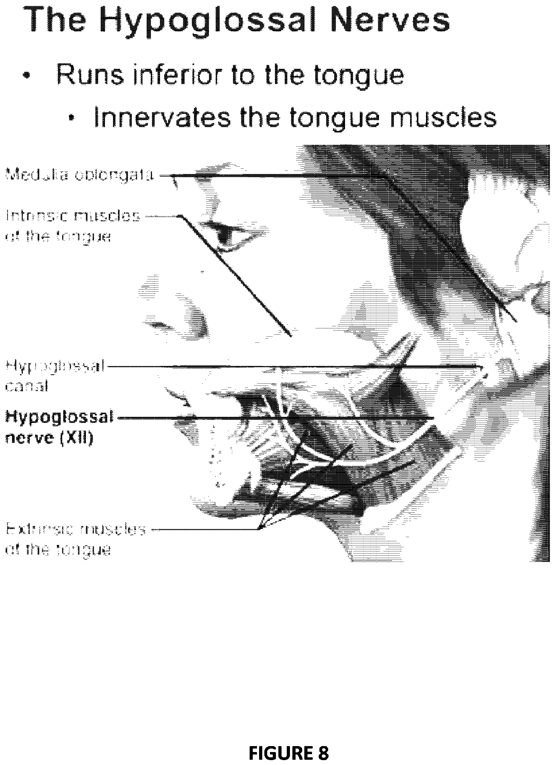

In one embodiment of a particular anatomical external stimulation with reference to the anatomical drawing of Figure 8, there is provided an external stimulation of the tongue in order to improve openness of the airway and effect improved breathing or reduce snoring or assist other breathing related disorders. In another form, the invention includes an external stimulation of the hypoglossal nerve region around the submental area tongue in order to improve openness of the airway. In still another form the invention there is provided a variable external stimulation of the tongue in order to retain effectiveness of improving openness of the airway.

As shown in Figs. 6, 7 and 18, there is shown the sensors and location and mode of the sensors and the stimulant and mode and location of the stimulant that can be enacted based on the sensor. These stimulants and location can include acoustic stimulant located at or near the ear, touch or acoustic or electric pulse stimulant at or near the chin, head, finger foot. In a particularly preferred form that is believed beneficial is an external stimulant to the hypoglossal nerve using a TENS system.

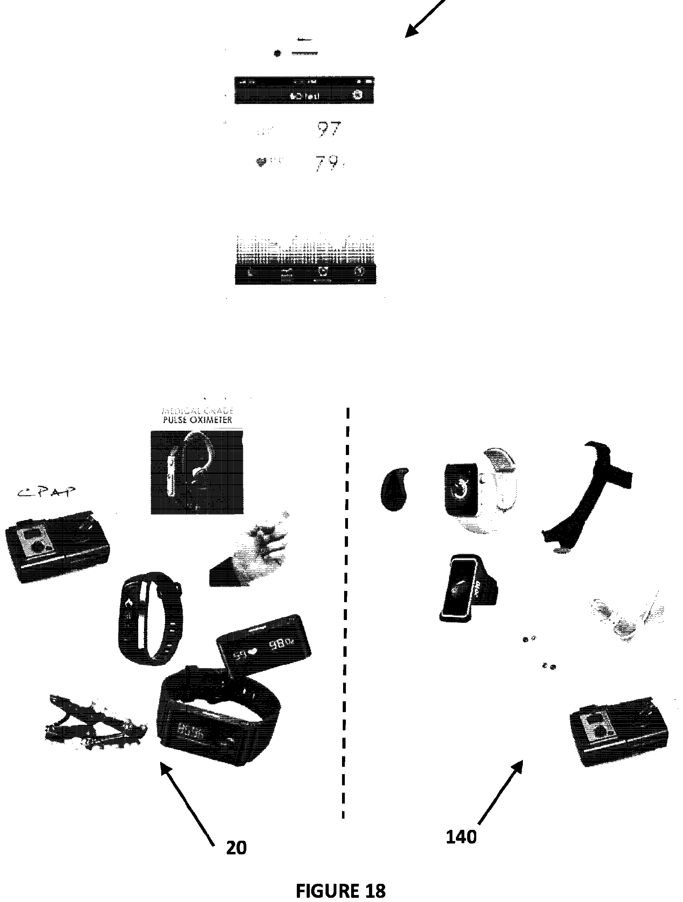

As shown in Figure 18 In a particular preferred form, there is provided a range of technologies that can be combined into this system to allow for aiding management of a breathing related disorder which includes at least one sensor for sensing at least one characteristic of a breathing related disorder; a predefined operative framework having at least one external stimulator positionable relative to a user for providing an effective external stimulation option to the user a control means for providing to the at least one topical stimulator a predefined effective treatment range of the at least one topical stimulator positionable on the user in managing control of a breathing related disorder of the user; wherein the predefined effective treatment range and control aids management of a breathing related disorder by an effective external stimulation.

### Multiple Sensors

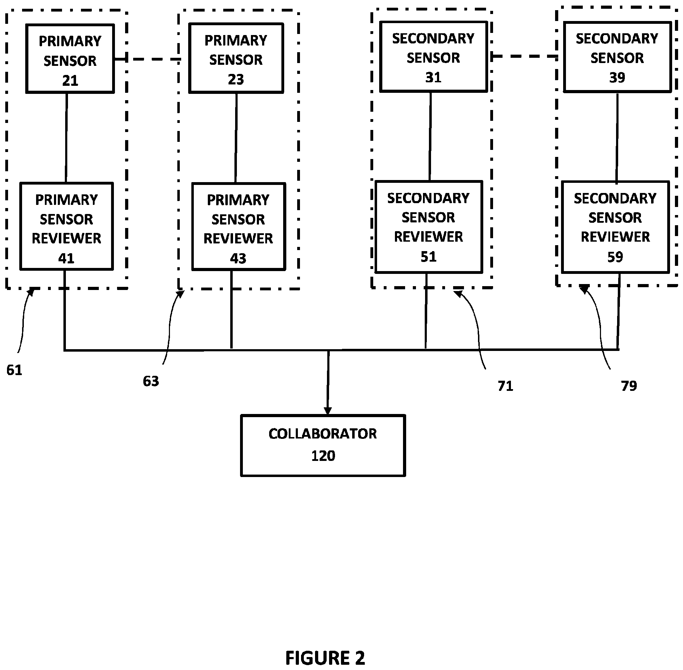

Referring to Fig. 2 there is shown the use of multiple sensors. This in one advantageous form includes a primary sensor 20 and a secondary sensor 30. In this way multiple sensors deliver uplifted accuracy even if the primary sensor is the more accurate or detailed or more suitable for the user. However, the invention allows the ability to maintain only an external sensing of the user rather than the invasiveness prior art having a device with invasive internal sensors, which can only be used under strict medical or professional supervision. This prior art immediately detracted from the wish for the user to use the device and will then increase the risk of ill effects of breathing related disorder because there is less detection. Therefore, the present system ensures by the use of external sensors that no detraction occurs and the user is extremely willing to use it to over extended times.

The complication with external sensors is that there are increases in false positive readings in that sensed data could incorrectly advise of sensed data that would be expected to indicate an occurrence of ill effect of a breathing related disorder. If the stimulator is then applied the user is affected or woken or alarmed for incorrect reasonings. It is therefore particularly beneficial to have a primary sensor 21 with its secondary sensor reviewer 41 as well as a secondary sensor 31 with its secondary sensor reviewer 51.

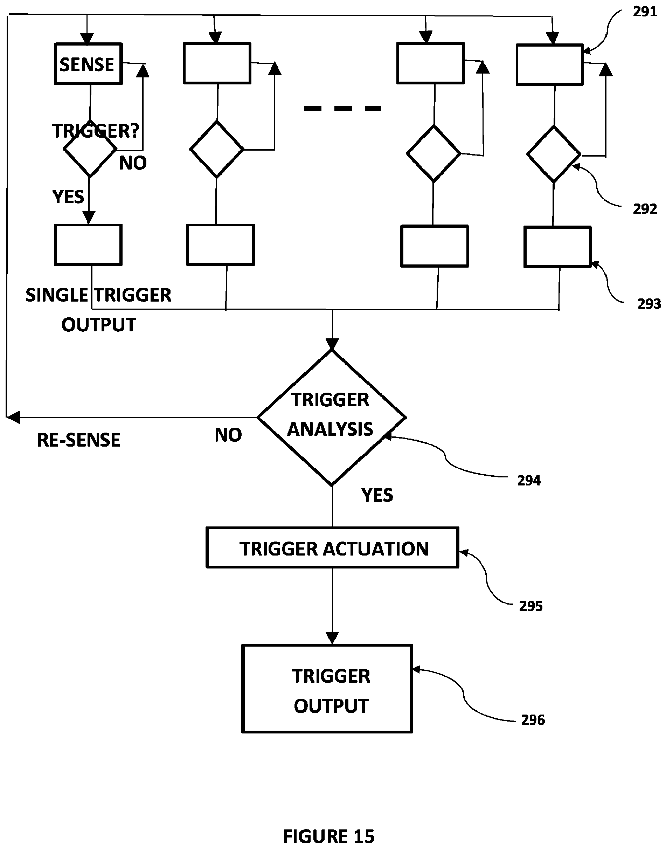

The first effect therefore as shown in Fig. 2 is that the primary sensor 21 has its sensed data reviewed by the primary sensor reviewer 41 and if the reviewer automatically assesses and compares the sensed data with operating conditions that should indicate an ill effect then a single sensor trigger provides a single sensor trigger output to the collaborator 120 as part of the operative framework 15. This does not yet result in a trigger as shown in Fig 15 but instead needs to be supported by a further sensor.

Due to the difference in effectiveness of sensors it is considered that they would be categorized into primary and secondary sensors and that in the plurality of sensors used there is at least one primary sensor.

Looking at Fig 15 there is a sensing at step 291 by each of the plurality of sensors used and if in the single sensor review at step 292 there is automatic assessment that the sensed data falls outside acceptable levels then the reviewer at step 293 issues the single trigger output. At step 294 the collaborator receives multiple sensed data or not and if over a particular time period receives two or more single

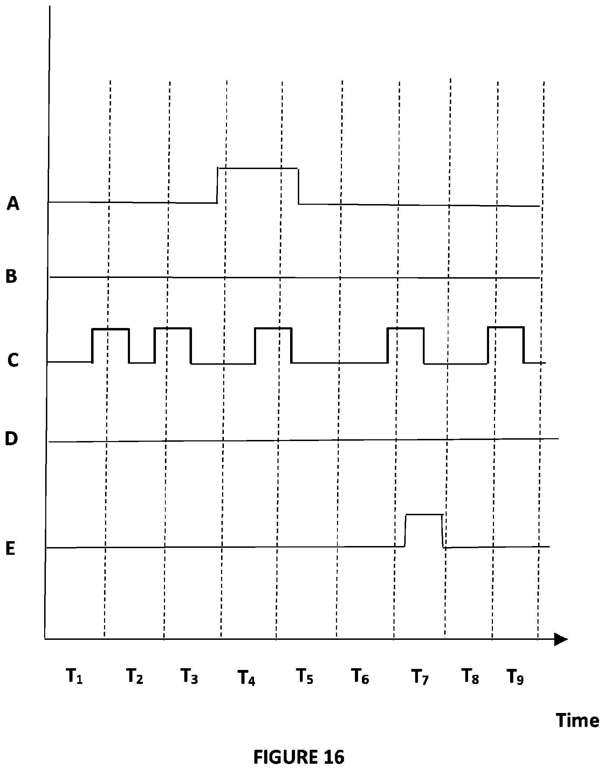

Fig. 16 is illustrative at the time period receipt of multiple signals or not from the single sensors. Clearly if sensor C was the only sensor then the user would receive 5 alarms over the period of T1 to T9 but it is expected that a number of those are false positives. By having Sensor C with Sensor A then it is only during T4 that there is two single sensor triggers and therefore trigger actuation step 295 will only occur at T4.

### Similarly as shown if sensor B or D was used then no

Primary sensors can be one of the following:
- 02 Oxygen. (Levels below 92% Oxygen in your blood is a sign of a breathing problem.
- Pulse / Heart Rate
- Blood pressure

Secondary sensors can be:
- Geo-Positioning (Upright, Laying down, Moving Activity)
- Temperature
- Diaphragm movement.
- Steps & Activity & Floors Climbed
- Calories Burned
- Sleep Tracking
- Sleep Stages (Light, Deep, REM)

Referring to Pulse Oximetry Overview there is a direct correlation between Oxygen levels and Heart rate for Apnea related chronic conditions. This can also be extended to people suffering from snoring.

You will also see pulse oximetry being used in a number of clinical settings, such as in the emergency department and in the operating theatre, to assess and monitor patents, so it is important to know about its uses and limitations, as well as how to interpret the readings that it produces.

The fundamental principle behind pulse oximetry is that when you shine light of a certain wavelength at molecules of oxygenated and deoxygenated haemoglobin differing amounts of light are absorbed by these molecules. So, if you place a light source emitting these specific wavelengths of light on one side of the finger and a sensor that detects these wavelengths of light on the other side, one can measure the amount of light being absorbed within the tissue by oxygenated and deoxygenate haemoglobin. The reading that is produced (the Sp02) represents the percentage of oxygenated haemoglobin present as a proportion of the total amount of haemoglobin detected. So, a reading of 92% means that the pulse oximeter has detected that 92% of the haemoglobin molecules sampled are carrying oxygen and 8% are deoxygenated molecules. Pulse oximeters are designed to provide readings on haemoglobin molecules that are travelling in a pulsatile manner, so the reading represents the situation that exists in the arterial circulation. You will see pulse oximetry probes being applied to fingers and toes and also to hands, feet and earlobes in babies

It can be that the user or more particularly the user under review by a medical practitioner, completes a Questionnaire to obtain a high-level snapshot of user's position. This can be used to assess various risk rating(s) and recommend number of sensors to use.

The key input functions can have readings which can be used in any combination by the user as part of their monitoring configuration, which is fully configurable by the user or by their medical practitioner.

By the importance of Sensor C being a primary sensor, if it is used in combination with a secondary sensor B or D and those sensors failed to pick up any trigger point by the time the C sensor has triggered 3 times then the 3rd trigger of the primary sensor advises of the multiple single sensor trigger and warns for testing or changing of the secondary sensors.

The great benefit of the system is that Sensor B and D which might be defective or not suitable or effective for the particular user can readily be replaced by connection to different sensor such as Sensor A or E and thereby the system again functions to its maximum effectiveness while eliminating false negatives.

Apart from the idea that the second sensor would need to have sensed data that in itself would be reviewed by its sensor reviewer to determine if trigger for ill effect, and that therefore there are two triggers and avoidance of false negatives, a substantial benefit of the plurality of sensors is that the sensor can be operating with less sensitivity and still be effective in combination. In particular the pick-up of the sensor can be operating at 80% but is amplified. This in effect increases the chance of false negatives by a single sensor but that is offset by the need for the second or multiple sensors to trigger to provide an actual trigger event. This provides greater overall accuracy from external non-invasive sensors without having to use more accurate or more intrusive or internal sensors.

In effect therefore the plurality of sensors allows a plurality of the at least one external sensor and a control means for receiving to the predefined operative framework a predefined effective operative range of each of the plurality of the at least one external sensor; wherein sensing by at least two of the plurality of the at least one external sensor within the predefined effective treatment range and control aids early detection and management of a breathing related disorder by an effective sensing by the at least one sensor.

### Modules

A particular advantageous version of the invention includes the use of modules in order for the user, the medical professional or advisor to select and connect modules that are particularly effective to you.

### 1. Input Selectable Sensor Modules

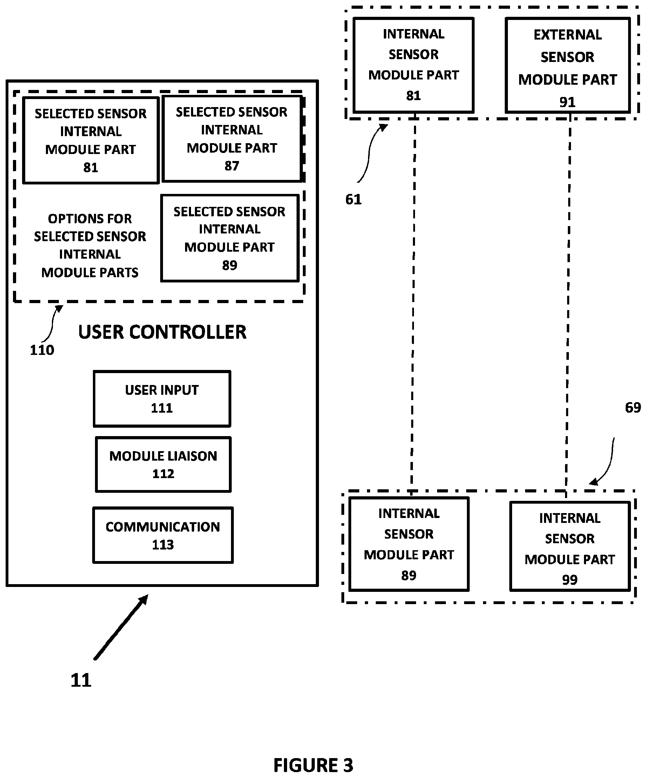

Referring to Figs 3, 4 and 5 there is shown an embodiment of a system for aiding early detection and management of breathing related disorders wherein the user controller includes connection with one or more input selectable sensor modules providing the at least one sensor.

The user controller 1 1 has a user input 111 for receiving input of selection or usage requirements from the user. However the primary input is from sensors and has the user controller 11 having location 1 10, for options for receipt physically or digitally of selected sensor internal module parts.

The user can select a sensor 61 to 69 and each sensor will have its internal sensor module part 81 to 89 and corresponding external sensor module part 91. These parts can be integral but generally will be in wireless communication.

The one or more input selectable modules includes a selected sensor internal module part for connection with the user controller and an external sensor module part for location on an external body location and for providing sensed data to the selected sensor internal module part.

The internal module parts 81 , 87, 89 of the selected sensors 61 , 67, 69 will be downloadable or insertable into the user controller and can communicate through the module liaison 112 as required with the rest of the operative framework 15 of the user controller 1 1 or with external connections through the communication 113.

The external sensor module part 91 to 99 is positionable to sense at at least one or more of the following external body sensor locations of:
Head
   - throat
   - nose
   - mouth
▪ Ear
Wrist
   - Finger
   - Foot/limb

The external sensor module part is selected to sense according to at least one of the following modes of:
- Pulse / heart rate
- Oximetry / 02 levels
- Temperature
- Body movement • breath

It can therefore be that a user (which can be under the guidance of a medical practitioner) undertakes a selection of primary sensor module 61 and secondary sensor module 71 and these are installed into the user controller 1 1 with an internal part 81 in communication with an external part 91 that is attached externally to the user when in use.

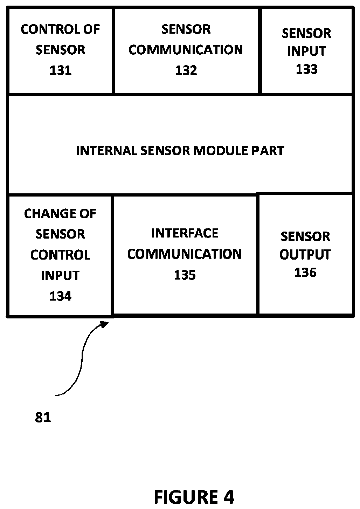

The internal sensor module part 81 , as shown in Fig. 4 includes the primary element of the sensor input 133 for receiving from the corresponding external sensor module part 91 which is detecting the required characteristic at the required location and under the required mode. This communication can be directly between the internal and external parts or through the communication interface 135 to the communication part 1 13 of the user controller.

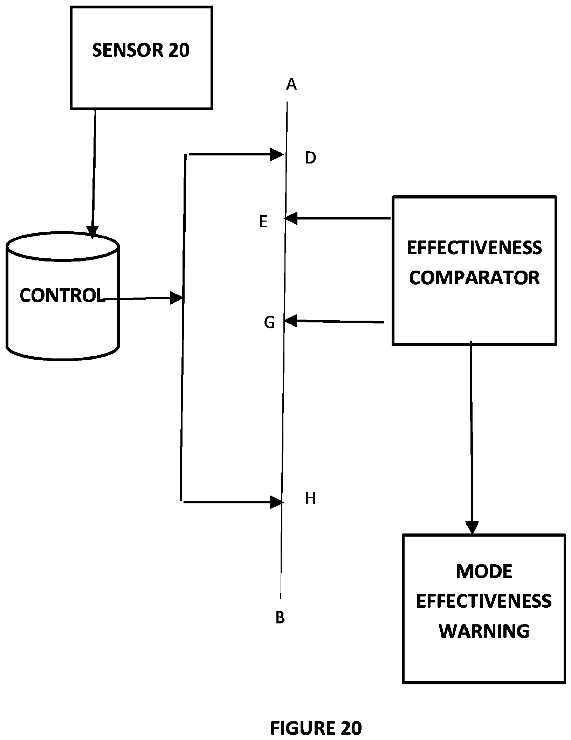

The internal sensor module part 81 further includes a control of sensor part 131 for providing the required operating settings and control mechanisms of the external sensor module part 91. This control section 131 can include inherent controls that are predefined in order to operate the sensor and can receive changes, elaborations, calibrations or fine tuning from a change of sensor control input 134. This input can receive input directly through user input 111 of the user controller from the user or directly or indirectly from an external set-up control 52 such as downloadable from an updatable external website. There also can be input directly from medical control 53 such as updates on effectiveness of particular sensing of characteristics for indicating particular breathing disorders or adjustments by the medical practitioner due to the actual patient characteristics or requirements. Further the change of sensor control input 134 can receive feedback control information such as in Fig. 20 so that the operation of the sensor can be self-calibrated. This is of particular benefit due to the external use of sensor and variability of sensor sensitivity in that form of use.

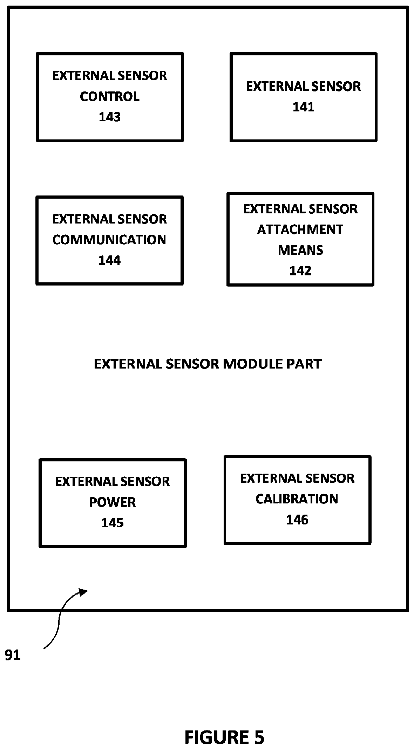

The external sensor module part 91 of Fig. 5 also has its component parts including the primary external sensor 141 that is placed in the required position with the required mode. This can be retained in place by the external sensor attachment means 142 that could be a strap, belt, adhesive, or other attachment means.

The external sensor 141 can be controlled by the external sensor control 143 that can be in communication with the internal sensor communication module through the external sensor communication 144. Preferably this is wireless so as to not limit the positioning of the external sensor module part 91 with the user controller but could have wired connection or be integral. The external module 91 has an external sensor power 145 that can be due to battery, wireless power, wired power, solar, movement or otherwise powered. Further the operation of the external sensor can be calibrated by its direct sensing and review or through the further processing done separately from the external module such as in the user controller, in the internal sensor module part or further afield in the remote website.

It can be seen that having sensor modules and particularly with the incorporation of internal sensor module parts and external sensor module parts the device is particularly effective in providing a more effective application of a plurality of external sensors without wires extending everywhere and with coordination with single user controller while still being networked for medical assessment and medical control.

### 2. Output Selectable Stimulator Modules

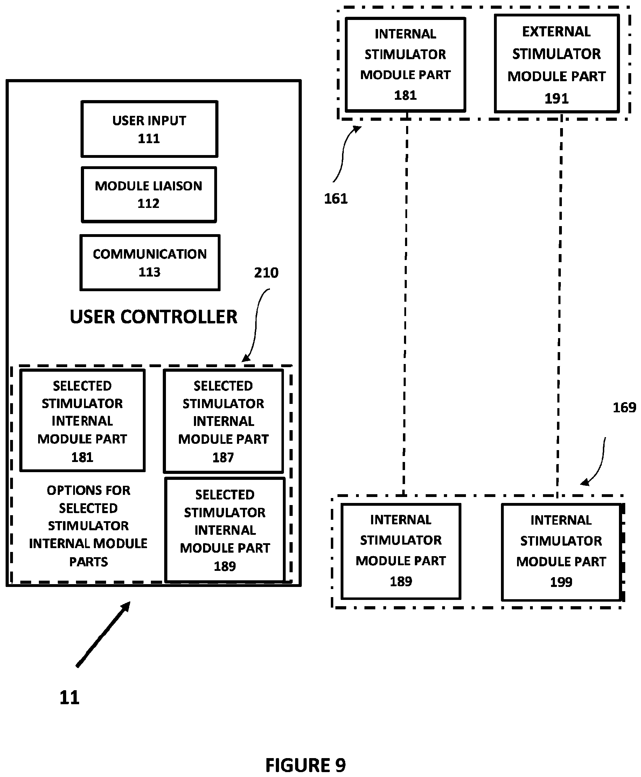

Referring to Figs 9, 10 and 11 there is shown an embodiment of a system for aiding early detection and management of breathing related disorders wherein the user controller includes connection with one or more output selectable stimulator modules providing the at least one external stimulator.

The one or more output selectable stimulator modules includes a selected stimulator internal module part for connection with the user controller and an external stimulator module part for location on an external body location and for providing stimulation to the selected stimulator external module part.

The user controller 1 1 has a user input 111 for receiving input of selection or usage requirements from the user. However the primary input is from sensors and has the user controller 11 having location 1 10, for options for receipt physically or digitally of selected sensor internal module parts.

The user can select a stimulator 181 to 189 and each sensor will have its internal sensor module part 191 to 199 and corresponding external sensor module part 161. These parts can be integral but generally will be in wireless communication.

The one or more input selectable modules includes a selected stimulator internal module part for connection with the user controller and an external stimulator module part for location on an external body location and for providing stimulation via the selected stimulation external module part.

The internal module parts 181 , 187, 189 of the selected stimulators 161 , 167, 169 will be downloadable or insertable into the user controller and can communicate through the module liaison 112 as required with the rest of the operative framework 15 of the user controller 11 or with external connections through the communication 113.

The external stimulator module part 191 to 199 is positionable to stimulate at at least one or more of the following external body sensor locations of:
- Head
- throat
- nose
- mouth
- Ear
- Wrist
- Finger
- Foot/limb

The external stimulator module part 191 to 199 is selected to sense according to at least one of the following modes of:
Electrical pulse TENS/ EMS
   - Sound / Audio
   - Touch / Physical
   - Vibration
Light
   - Other party notification
(SMS/text alert message or integral app.)

It can therefore be that a user (which can be under the guidance of a medical practitioner) undertakes a selection of stimulation modules 181 and these are installed into the user controller 11 with an internal part 181 in communication with an external part 191 that is attached externally to the user when in use.

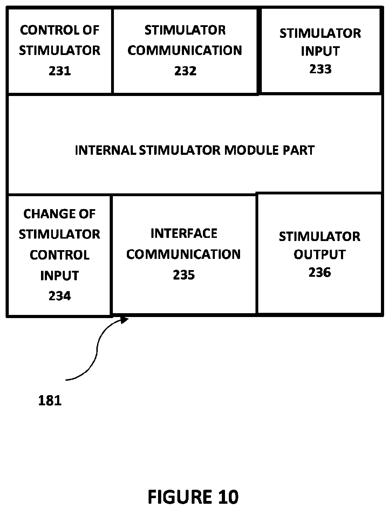

The internal stimulation module part 181 , as shown in Fig. 10 includes the primary element of the stimulation input 233 for receiving from the corresponding external stimulation module part 191 which is detecting the required characteristic at the required location and under the required mode. This communication can be directly between the internal and external parts or through the communication interface 235 to the communication part 213 of the user controller.

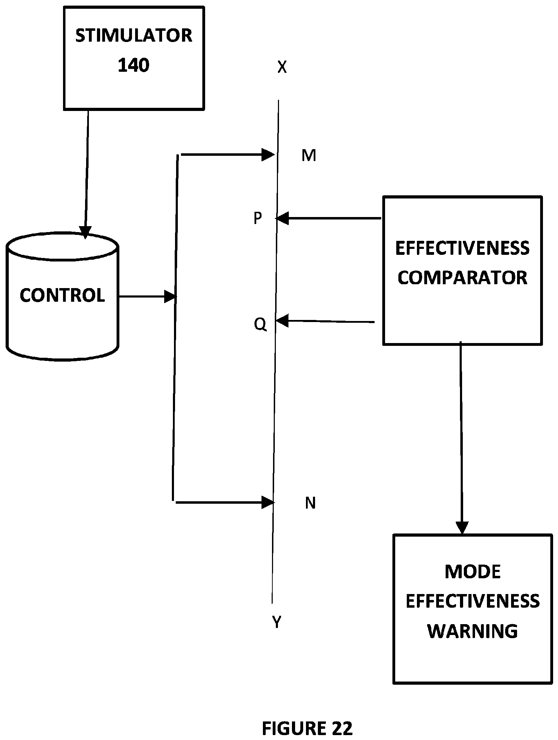

The internal stimulation module part 181 further includes a control of stimulation part 231 for providing the required operating settings and control mechanisms of the external stimulation module part 191. This control section 231 can include inherent controls that are predefined in order to operate the stimulation and can receive changes, elaborations, calibrations or fine tuning from a change of s stimulation control input 234. This input can receive input directly through user input 1 11 of the user controller from the user or directly or indirectly from an external setup control 52 such as downloadable from an updatable external website. There also can be input directly from medical control 53 such as updates on effectiveness of particular stimulation of characteristics for indicating particular breathing disorders or adjustments by the medical practitioner due to the actual patient characteristics or requirements. Further the change of stimulation control input 234 can receive feedback control information such as in Fig. 22 so that the operation of the stimulation can be self-calibrated. This is of particular benefit due to the external use of stimulation and variability of stimulation sensitivity in that form of use.

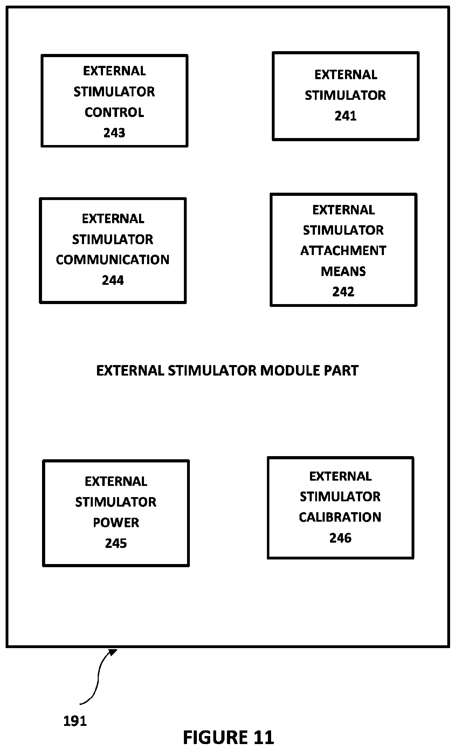

The external stimulation module part 191 of Fig. 11 also has its component parts including the primary external stimulation 241 that is placed in the required position with the required mode. This can be retained in place by the external stimulation attachment means 242 that could be a strap, belt, adhesive, or other attachment means.

The external stimulation 241 can be controlled by the external stimulation control 243 that can be in communication with the internal stimulation communication module through the external stimulation communication 244. Preferably this is wireless so as to not limit the positioning of the external stimulation module part 191 with the user controller but could have wired connection or be integral. The external module 191 has an external stimulation power 245 that can be due to battery, wireless power, wired power, solar, movement or otherwise powered. Further the operation of the external stimulation can be calibrated by its direct sensing and review or through the further processing done separately from the external module such as in the user controller, in the internal stimulation module part or further afield in the remote website.

It can be seen that having stimulation modules and particularly with the incorporation of internal stimulation module parts and external stimulation module parts the device is particularly effective in providing a more effective application of a plurality of external stimulation without wires extending everywhere and with coordination with single user controller while still being networked for medical assessment and medical control.

It can therefore be that a user or under the guidance of your medical practitioner there is a selection of stimulator module or modules and these are installed into the user controller with an internal part in communication with an external part that is attached externally to the user when in use.

Looking at components in further detail:

### 1. Input Devices:

The input devices can be a sensor connectable external to the user for providing characteristics of the user that relate to the effective breathing of the user. Such sensors can be undertaking sensing of the ear, foot, or wrist or other extremity providing access to vital characteristics.

In the form of a Smart Ear device acting as an input device, it can send the following features and functions to the Health App:
i) Collected heart rate and blood oxygen (Sp02) at predefined time intervals in real time to identify when the user is having a Sleep Apnea episode. ii) And Utilizing gyroscope technology, accelerometer or similar identifies when the user is sleeping too long on their back.

The Smart Foot device, acting as an input device, it can send the following features and functions to the Health App:
i) Collect heart rate and blood oxygen (Sp02) at predefined time intervals in real time to assist in identifying when the user is having a Sleep Apnea episode.

The Smart Band device, acting as an input device, can send the following features and functions to the Health App:
i) Collect blood pressure, heart rate and blood oxygen (Sp02) at predefined time intervals in real time, indicating when the user is having a Sleep Apnea episode. ii) Record sport data, health fitness activities and provide reminders.

### 2. Smart devices and the Health App:

Various 'smart' devices can be used, which can connect with smart devices using smart technology including computers, tablets, phones, watches, wearable devices, or other Internet of Things (IOT) supporting Windows, Apple & Android. Data is collected from the smart input devices using Bluetooth, WiFi or other wireless technologies.

Users use the free Health App to setup and configure all input/output options to accommodate their specific requirements. The Health App manages all inputs and sends Output actions to the devices to inform the user.

The Input devices can also serve as the Output devices performing multiple functions.

### 3. Output Devices:

The Output devices receive notifications from the Health App via Bluetooth WiFi etc. for activating the external stimulator positionable relative to a user for providing an effective external stimulation option or external notification option to the user. The Output devices convert these incoming signals to specific actions and directly or indirectly informing or enticing or urging consciously, subconsciously or automatically the user to take preconfigured actions.

Clearly the most effective external stimulant is one that urges the user to automatically undertake an action to effect an alteration to the user's breathing.

There are various Output devices available to best accommodate the user's preferences and requirements. Output devices can be used to help manage sleep apnea, snoring and SI DS symptoms.

### 3A Output Devices - The TENS/EMS Device:

In using a TENS/EMS Device the device is fitted externally over the submental region directly under the user's chin. The device can be worn or fitted as part of a neck brace, chin strap, adhesive contact or similar.

In one form of TENS/EMS device a Head-Strap is fitted over the head, placing the "TENS" Stimulation-Device directly under the user's chin. The strap also holds the chin in a more optimal position, delivering additional benefits.

The "TENS7EMS stimulation-device delivers the following to stimulate the submental area (tongue muscles, hypoglossal nerves) to move and help improve user's breathing by delivering a very small vibration and/or electrical stimulation via the TENS/EMS device that will:
- notify the user to alter positions and/or take suitable steps
- to improve their breathing capability and increase oxygen flow.
- notify the user they are sleeping too long on their back,
- prompting them to move to their side.

### 3B Output Devices - The Smart Ear:

The SmartEar is fitted into the user ear. This device is a multipurpose device, acting as an Input and Output device in one.

The SmartEar delivers the following to help move the tongue muscles and help improve users breathing by delivering a very small vibration that will:
a) notify the user to alter positions and/or take suitable steps
   to improve their breathing capability and increase oxygen flow.
b) notify the user via a Vibration or Sound they are sleeping too long on their back, prompting them to move to their side.
c) Enables you to receive/make calls, listen to music from the Smart device.
d) notify you when the smart device is low on battery.

### 3C Output Devices - The SmartBand:

The SmartBand is fitted onto the user wrist. This device is a multipurpose device, acting as an Input and Output device in one. It is also waterproof (IP67)

The SmartBand delivers the following to help move the tongue muscles and help improve users breathing by delivering a very small vibration that will: a) notify the user to alter positions and/or take suitable steps to improve their breathing capability and increase oxygen flow.
b) notify the user via a Vibration or Sound they are sleeping too long on their back, prompting them to move to their side.

Other key functions include:
- Enables you to receive/make calls, listen to music from the Smart device.
- notify you when the smart device is low on battery.
- Receive message from Facebook and Twitter
- Select from 8 languages.
- Monitor users sleep quality
- Receive sport data and incoming reminders

### 3D Output Devices - The SmartFoot:

The SmartFoot is fitted over the user's foot. This device is a multipurpose device, acting as an Input and Output device in one.

The SmartFoot delivers the following to help move the tongue muscles and help improve users breathing by delivering a very small vibration that will:
a) notify the user to alter positions and/or take suitable steps
   to improve their breathing capability and increase oxygen flow.
b) notify the user via a Vibration or Sound they are sleeping too long on their back, prompting them to move to their side.
c) notify you when the smart device is low on battery.

### 3E Output Devices - TENS and EMS technology

Hypoglossal nerve stimulation (HNS) has been undertaken primarily by invasive internal circumferential nerve cuff electrode, a stimulation lead and an implantable pulse generator.

There is use of TENS devices. TENS - stands for "Transcutaneous Electrical Nerve Stimulation", a non-invasive drug free method used by physical therapists and prescribed by Doctors for over 30 years. TENS consists of a device that transmits low- level electrical impulses to the body. A mild electrical current travels through your skin and along your nerve fibres which may cause a warm, tingling sensation.

However, a problem had arisen from the previous observations, that such stimulating of a single protrusor muscle could result in the antagonistic activation of other neck and tongue muscles which could evoke an antagonistic effect on airway patency. However, stimulating the hypoglossal nerve could also lead to the stimulation of multiple tongue muscles, which could lead to a synergistic effect and a favorable effect.

By use of external stimulator there is a sifter more effective synergistic action on the tongue muscles and thereby improved breathing. TENS unit is designed to provide nerve stimulation, placing the electrode pads correctly on a muscle can cause a strong muscular contraction.

EMS stands for electrical muscle stimulation. So, you will be simulating a completely natural procedure. Since, for a muscle to move, it has to receive electrical stimulation via the nerve pathways. In an EMS treatment, this stimulation comes from a handy electrical pulsing device in the right strength and frequency via electrodes on the skin. By means of various current strengths, frequencies and intervals you can use your TENS-EMS device for selective muscle activation or relaxation.

For Correct Muscle Stimulation Through EMS, current devices allow you to choose between various programs and settings. Synchronous (S) and asynchronous (A): With synchronous programs, the stimulation is carried out simultaneously on all available channels, while with asynchronous programs this happens with a time delay. This, for example, allows a particularly thorough or a particularly gentle stimulation to be achieved. Clearly for external stimulation of the hypoglossal nerve at the rear of the oral cavity at the rear of the tongue a gentle but pulsed stimulation is effective.

Frequency is significant for most EMS applications and is given in Hertz (Hz). When choosing the frequency, you should take into account that there are individual differences.

Low frequencies (no higher than about 18 Hz) will mainly activate the slower reacting red muscle fibres. Higher frequencies between 30 and 50 Hz stimulate the fast- contracting white muscle fibres.

With frequencies of over 50 Hz, the muscle is deliberately overtaxed and can thus be forced into muscle hypertrophy (muscle build-up). The interval between the sessions must be correctly chosen so that the muscle has enough time to regenerate.

The pulse width or pulse duration is given in microseconds (µε). With longer pulses, the effect goes deeper and is mainly suited to larger muscles. For smaller muscles, the duration will remain below 200 µε. Some programs offer a varying pulse duration to stimulate the muscle even more intensively.

Cold muscles should never be put under full strain even with EMS. That is why modern EMS devices ensure that muscles are gently warmed and supplied with blood by pre-tensioning. For the untrained, the minimum time for this is 2 seconds.

The contraction time (ON) is chosen to be through numerous, relatively short (4-6 seconds) stimulations. Pauses are usually at least twice as long as the contraction time.

Modern TENS/EMS devices operate almost exclusively with biphasic pulses, which are gentler on the skin of the user. This means every current pulse is followed by a phase with a negative counter-oscillation below the zero line.

General settings can be:
- Lower Frequency: Max. 15-18 Hz
- Short Contraction Duration: 4-6 seconds
- Short Pause Time: 3-6 seconds
- Small Muscle: Low pulse width (50 - 100 µε)

### Variable Usage

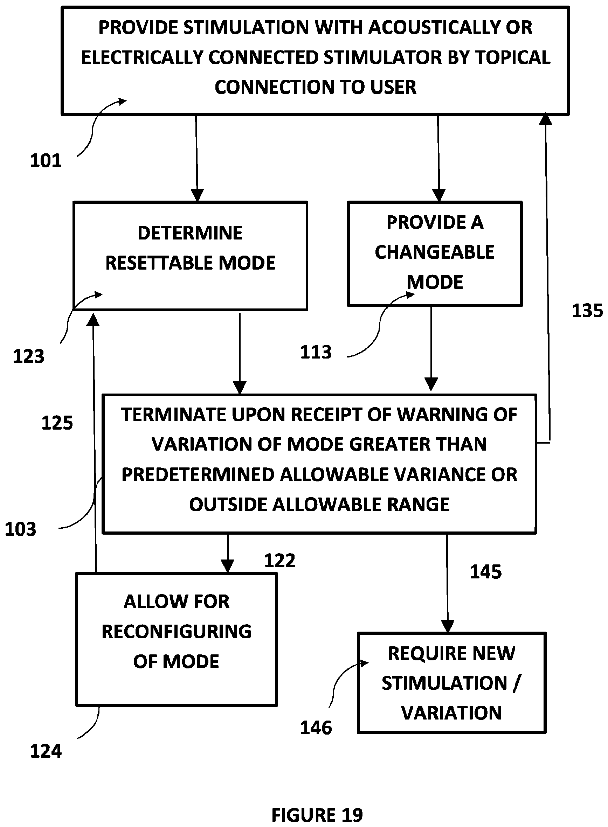

Referring to Figs. 19 to 23 there is shown the self-modifying operation of the sensors and external stimulators by feedback and control in a variable system for aiding management of a breathing related disorder. This is particularly effective in ensuring that the body does not become acclimatised to the same stimulant and therefore becomes less effective.

Referring to Fig. 19, in one embodiment of a variable usage in which the predefined operative framework 15 is in receivable connection to the sensors 20 locatable on the user to receive information from the sensors about the sensed characteristic of a breathing related disorder and the predefined operative framework 15 has also operable connection to at least one external stimulator of a plurality of stimulators 40 positionable relative to a user for providing an effective external stimulation option to the user.

In operation of such variable usage, in step 101 there is provided stimulation acoustically or electrically or physically by an external stimulator 140 being positioned topically on the user.

In step 113 there is provided a changeable mode. This changeable mode can use a particularly effective mechanism of having a predefined operative framework with a plurality of external stimulators that are from more than one category. Therefore, the predefined operative framework identifying a plurality of stimulation options includes a plurality of categories or selection of at least one stimulation option in at least each of the plurality of categories by the user in their respective user input and wherein the categories of stimulation options include a plurality of:
a) Stimulation option category;
b) Adjusted stimulation option category; and
c) Adjusting form of stimulation from a stimulation in the Stimulation option category

The predefined operative framework identifying a plurality of stimulation options includes a plurality of categories includes a plurality of the stimulation option categories selected from:
a) Foot;
b) Wrist;
c) Ear;
d) Head;

The categories of stimulator can be selected from:
i) Acoustic which can give an audible alarm; ii) Physical which can give a prod/vibration/displacement at the site of the hypoglossal nerves, the ears, toes, or fingers; or
iii) Electrical pulse which can give a short circuit to the hypoglossal nerves to activate tongue retraction and to help improve breathing.

In providing the changeable mode step 113 there is the operation of the mode until step 103 of determining and warning of the variation of the mode of the sensor 20 and/or external stimulator 140 being greater than predetermined allowable variance or outside allowable range. This is further described later with reference to Figs. 10 to 13.

From the changeable mode step 1 13 being undertaken there are the two options of step 145 of a new mode or step 124 of altering of the present mode.

In step 145 the mode of external stimulator moves to requiring a new stimulation or variation of step 146. In this form, a different external stimulator 140 can be connected to the predefined operative framework 15. This can be by purchasing a different device or relocating to a different body part. Step 135 requires the return to initial step 101 after the new external stimulator 140 is connected, and set-up through the controls 51 , 52 and 53.

In the option of step 122 of moving to the step 124 of the adjusting form of stimulation. This adjustment step requites a determination step 123 of how to reset the mode to a new operational position with the same external stimulator but with a different operative mode from a stimulation in the Stimulation option category is selected from: a) Variable selected frequency,
b) magnitude,
c) period,
d) symmetry of stimulation.

Looking in more detail at the step 103 there can be operative self-assessment and adjustment of the sensors 20 in accordance with their operative conditions and predetermined medical operation conditions and personal preference operative conditions by the communication with the user control 51 , set-up control 52 and medical control 53. There also can be operative self-assessment and adjustment of the external stimulators 40 in accordance with their operative conditions and predetermined medical operation conditions and personal preference operative conditions by the communication with the user control 51 , set-up control 52 and medical control 53.

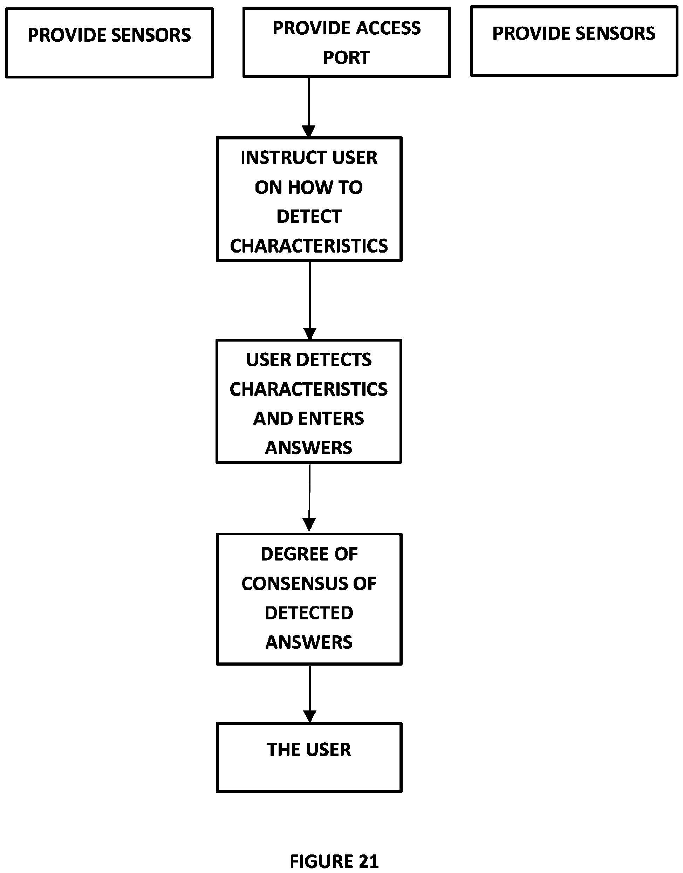

Referring to Figs. 20 and 21 there can be a particular sensor 20 that can operate between A and B. However, the control means provides an operative range between D and H that is predetermined so that it would provide the effective sensing by the sensor of a characteristic of the user's breathing and sensing of the effects an alteration to the user's breathing. The operation of the sensor can therefore be at E which is within the range of D to H on the A-B scale. However, by feedback of the sensors it can be determined if the effectiveness has slipped and needs change to G to re-enhance the effectiveness. Still further if the feedback shows the stimulator falling outside the effective range of D to H then a signal can be sent to the mode effectiveness warning. This can ensure that the variation of the effectiveness in the predefined sensor is immediately instigated. This can allow for change of sensor or for a totally different operating sensing range from A-B is chosen or a change in pick-up signal or change in intensity. It can also allow change of category of sensor.

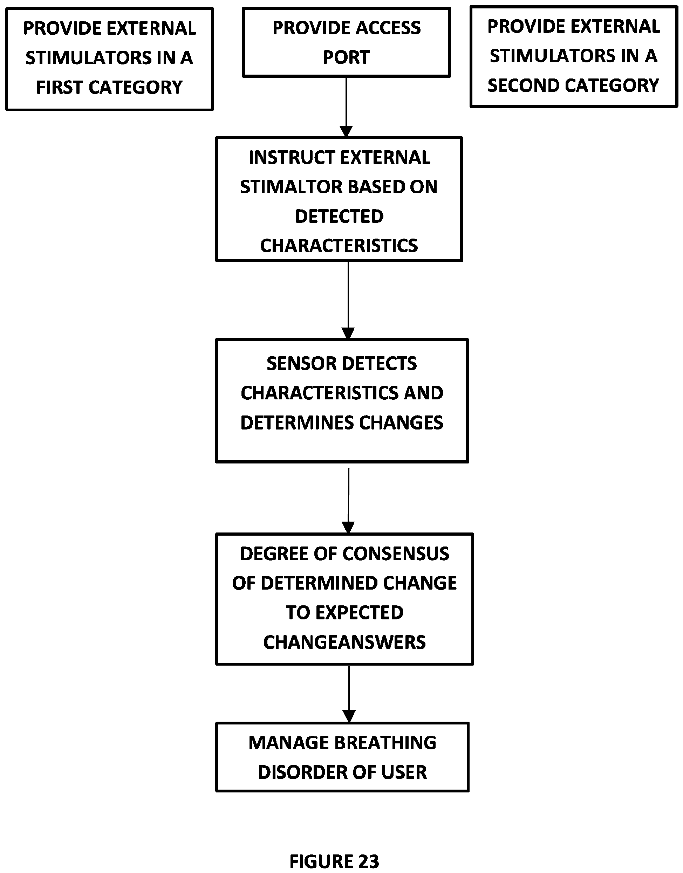

Referring to Figs. 22 and 23 there can be a particular external stimulator 140 that can operate between X and Y. However, the control means provides an operative range between M and N that is predetermined so that it would provide the effective external stimulation by the at least one external stimulator effects an alteration to the user's breathing. The operation of the stimulator can therefore be at P which is within the range of M to N on the X-Y scale. However, by feedback of the sensors it can be determined if the effectiveness has slipped and needs change to Q to re-enhance the effectiveness. Still further if the feedback shows the stimulator falling outside the effective range of M to N then a signal can be sent to the mode effectiveness warning. This can ensure that the variation of the selection in the predefined operative framework is immediately instigated. This can allow for change of mode of stimulator such that a totally different operating range from X-Y is chosen or a change in signal or change in intensity. It can also allow change of category of stimulator.

It can be seen that the system is built in order to overcome the propensity for the user to be de-sensitised and acclimatized to the external stimulant. Further the sensor effectiveness might change due to the sensed characteristic no longer being related as precisely. Recalibration can be automatically achieved by the reassessment of the sensed characteristic and its meaning for the particular user at that particular time by the feedback of sensing and stimulation and further sensing.

### EXAMPLE - SCENARIO A

In its simplest form, the Apnea user would go to bed along with the usual anxieties associated with anticipated breathing difficulties or in some cases complete lack of oxygen and the dreaded awakening or choking symptoms. These factors contribute in addition to the Apnea side effects, which include apprehension, anxiety, stress and insomnia, which in turn contributes to other health issues.

Whilst sleeping the simplest form of the management App would involve the Apnea recipient (user) to wear an input device on a preferred part of their body, based on personal preference e.g. wrist, foot, ear, etc.

This input device would take readings of the users' Oxygen levels via Oximetry and Pulse recordings.

The Input Device(s) measure concurrently and detect both the Oxygen changes and pulse level changes and prepares this data to be sent to the Apnea Management System.

The Apnea Management System receives data from the Input Device(s) via Wireless* technology and identifies pre-configured reductions in oxygen level with concurrent increase in pulse level, identifying the onset of symptoms which could contribute to an apnea episode and alert the user based on their settings.

The Apnea Management System would communicate via Wireless technology with the Output Device(s) via audible and/or vibrational methods. This alerting method will notify the user of a possible apnea episode and based on their settings, they can simply be gently notified (partially arouse). The alerting will increase in severity if user does not move and/or symptoms increase in severity.

The intended outcome here is to inform the user prior to experiencing the full onset of apnea symptoms to prevent short term (snoring effects) and long-term effects. (Wireless* = Wifi, Bluetooth and/or similar technologies) Note: The system can use both TENS and/or EMS

### EXAMPLE - SCENARIO B

Oxygen deprivation onset sets in when it reduces from 98%+ to 50-60% and increasing Pulse rate above typical resting levels. These are usually the two main indicators of Apnea that we can focus on enabling these readings to be communicated via Bluetooth to the Apnea Management System and subsequent Output management devices in either scenario A or B.

Whilst the sleeping scenario is the same as the above in terms of monitoring and the function of the input device(s), however in this scenario the technology used for the output device in this scenario will communicate via wireless with an output device located on the user's chin.

This output device is based on TENS technology and will be housed within a chin strap or self-adhesive, which would arouse the Hypoglossal region (nerve stimulation) to such an extent that the tongue muscle remains in a non-collapsed state averting apnea type symptoms, etc. These setting are fully controllable and customised by the user to ensure the TENS levels are set to an effective level specifically suited to them, which contributes to minimum sleep disruption and maximise apnea management.

As the human brain has the ability to eventually recognise nerve stimulus settings and could ignore the same stimulation over a period of usage. To ensure ongoing effectiveness the Apnea Management System will allow users to randomly select various ranges and depths of TENS stimulus.

The TENS output device also serves as a therapeutic benefit in allowing the user to enhance these muscles due to the ongoing stimulation. Effectively strengthening the muscles which could contribute to reduced apnea episodes.

The Apnea Management System will allow the user the option of selecting their preferred levels of nerve/muscle stimulation so as to fine tune minimum disruption from sound sleep and maximum management of Apnea symptoms and long-term health issues.

The Apnea Management System will monitor other aspects of their Health, enabling the user to record information which they can either use personally or provide to third parties. This data will also help their care practitioners to develop improved care plans. Examples include, ongoing Oxygen levels, pulse rates, steps, calories burnt. The user will also be able to enter other health data which will enable them and their doctor to correlate other health data in conjunctions with the above data, to provide a holistic assessment. This will include the ability to enter their weight, BMI, blood sugar level (Diabetics), carbs consumed, cholesterol, etc The Apnea Management System will have content feeds from various health sources and forums with social media integration.

The Apnea Management System will have an Alert Management System. This enables users to configure notifications to be sent to their partners, personal carers or nurses in the event their monitoring detects critical stages where the user has not responded and/or moved. This type of notification is useful if users have
▪ Their partner sleeping in another room.
▪ Their family member sleeping in another room.
▪ monitoring by a nurse within a hospital.
▪ monitoring by a loved one remotely.
▪ Enabling users to configure reports to be sent to their doctors (or care givers) to manage their progress.
▪ Enabling users to be informed that the Apnea Management System is running at full capacity with high regularity, recommending they consult their doctor for a CPAP assessment.

### EXAMPLE - SCENARIO C

In a multiple sensor form there can be input sensors of:
1. 02 Oxygen. (Levels below 92% Oxygen in your blood is a sign of a breathing problem.
2. Pulse / Heart Rate
3. Blood pressure
4. Geo-Positioning (Upright, Laying down, Moving Activity)
5. Temperature
6. Diaphragm movement.
7. Steps & Activity & Floors Climbed
8. Calories Burned
9. Sleep Tracking
10. Sleep Stages (Light, Deep, REM)

In Multiple sensors the approach can be:

| | | |
|---|---|---|
| Typical User | 1. O2 oxygen sensor | Most effective with two primary sensors but matched with secondary sensor with programmable weighting or selective single sensor trigger points as effective collaboration of multiple single sensor trigger points |
| | 2. Pulse/ heart rate sensor | |
| | 4. Geo Positioning Sensor | |
| Lone Sensor User | 1. O2 oxygen sensor | Effective single sensor that is non-invasive but is a primary sensor. |
| Simple watch Based Sensors | 2. Pulse/ heart rate sensor | Primary sensor and secondary sensor in readily created user controllers such as in digital watches |
| | [001] Geo Positioning Sensor | |
| Unstable O2 detection User | 3. Blood Pressure Diaphragm movement | Primary and secondary sensors that overcome particular problems of particular users that have problems with the effectiveness of above systems working for that user. |

In Multiple sensors the approach can be The Apnea App system will receive input data feed, in real time, from users watch device and other suitable devices, based on features and functions available to that device the user wants to utilise with their Apnea App.

Input functions 1 to 6 will contribute to an OUTPUT action to support the users Apnea profile. Input functions 7 to 10 will contribute to provide analysis into Users Dashboard in the Apnea App, providing analysis of these inputs, correlation of inputs 1 to 7 on how they may have varied and correlation to the Questionnaire responses they entered.

In this scenario, the user turns off Inputs 3 and 5, as they do not have Blood pressure issues and exercises in the evening regularly which could provide adverse temperature readings. User has also disables Input 6 as they do not have that input sensor.

Monitoring actions include:
- The Apnea App will monitor inputs 9 & 10 and track till user falls asleep.
- As User has identified in their questionnaire they are experiencing significant Apnea symptoms and difficult to sleep, they have further set Input 10 configuration to the 'Deep' level, to start taking Apnea preventative actions at this level as they don't easily reach the "REM" sleep stage.
- The Apnea App with monitor input 4 to ensure they are not upright and laying down.

This input works in parallel to input 9 & 10 to deliver accurate assessments.
▪ The Apnea App with monitor Inputs 1 & 2 in line with user settings.

### Low-Level-Output condition:

Once Inputs 1 & 2 detect and assess the criteria and configurations meets this condition, it will instigate and trigger the configured Output condition. Assess how much below trigger point did level go, how rapid was the decline and if its stabilised at the low level.

The Apnea App will monitor for re-occurrence event within a time cycle, as defined by user, and re-trigger Output condition. The Apnea App will monitor for frequency cycles on this condition and based on configuration by user, escalate up to a Mid-Level- Output condition. If configured by the user, a Low- Level-Output can include a TENS pulse (100Hz, 200ms) directly over the hypoglossal nerve.

### Mid-Level-Output condition:

Assess volume of Low-Level-Output conditions triggered during this sleep cycle, and/or, assess configured conditions requiring a Mid-Level-Output condition output to be triggered. (Note: A Mid-Level-Output is at a higher level than a Low-Level-Output.)

The Apnea App will monitor for re-occurrence event within a time cycle, as defined by user, and re-trigger Output condition. The Apnea App will monitor for frequency cycles on this condition and based on configuration by user, escalate up to a High-Level- Output condition. If configured by the user, a Mid-Level-Output condition can include a TENS multiple pulse (100Hz,225ms) directly over the hypoglossal nerve.

### High-Level-Output condition:

Assess volume of Mid-Level-Output conditions triggered during this sleep cycle, and/or, assess configured conditions requiring a High-Level-Output condition output to be triggered. (Note: A High-Level-Output is at a higher level than a Mid-Level- Output.)

The Apnea App will monitor for re-occurrence event within a time cycle, as defined by user, and re-trigger Output condition. The Apnea App will monitor for frequency cycles on this condition and based on configuration by user, escalate final Duress-Output to awaken the user. The Duress-Output is configured by user which can include one or more of the following:
- Continuously vibrate Users wrist device and/or other configured device(s)
- Continuously produce an audible tone on Users wrist device and/or other configured device(s)
- Call users preconfigured caller list, family, medical assist, etc.
- Send SMS/email alerts and/or other similar event notifications to preconfigured parties who may be setup to monitor user, which may have Dashboard setup.

If configured by the user, a High-Level-Output can include a TENS multiple pulse and combination (100Hz, 250ms) directly over the hypoglossal nerve.

Based on User configuration and amount of Apnea App stimulations to help prevent/manage Apnea episodes, the system will inform user to seek alternative (CPAP) options via appropriate consultation. Record all output actions taken for future review.

In this scenario, the user is only using Inputs 1 (02 Oxygen) and 4 (Geo-Positioning). The Apnea App will monitor inputs 9 & 10 and tack till user falls asleep. Once user is sleeping the Apnea App will monitor Input 1 (02 Oxygen) level and when it drops below the configured value of 85%, it will then check input 4 (Geo-Positioning)

Low- Level-Output condition: If the User is laying on their back, they will be notified via the configured Output settings to move and lay on their side.

### Mid-Level-Output condition: Not enabled, use has disabled.

High-Level-Output condition: Enabled to wake the user if 3 × Low-Level-Input conditions occur within 5 min and there is no action.

In this scenario, the user can use Key Output Devices including:
▪ Wrist strap/watch
• Other ear, nose, audio prompts, neck, arm, and other body part devices.
• Technical devices able to integrate with Apnea App System
• Smart phones, watches, tablets, laptops, notepads, computers, smart speakers capable of providing configurable/automated responses to user, etc. • Wire or wireless/Bluetooth technology or similar.

Key OUTPUT functions are provide the Output functions, which are configurable key output devices that can be used in any combination by the user as part of their management plan, which is fully configurable by the user.

Output Devices produce various actions/output, which can include vibration, audible sounds, other configurable Output Device capability, notifications (e.g. emails, calls, push-notifications, social media integration, hospital/medical systems, etc.) stimulation (e.g. TENs, electrical impulse to key areas, including the Hypoglossal Nerve, Neck or other approved areas)

The Apnea App HealthTrack Dashboard will correlate Apnea triggers, Output events and correlate all inputs to display a Dashboard that representation of all factors. For example, the User will be able to see in the Dashboard that since they have lost xKgs in weight and walking 10,000 steps per day, their Apnea episode volume/count is trending down and/or the volume of High-Level-Output conditions is reduced by 20%. The HealthTrack Dashboard will also represent the opposite outcomes if the data supports that.

The Apnea App can determine Low (Mild), Med (Moderate), or High (Severe), depending on the number of times in an hour the users breathing stops (Apnea) or becomes very shallow (hypopnea).
▪ Apnea episodes may occur from 5 to 100 times an hour.
▪ More than 5 Apnea's per hour is abnormal.
▪ 5-10 per hours can be considered Low (Mild) sleep Apnea.
▪ 10-30 per hours can be considered Med (Moderate) sleep Apnea.
▪ More than 30-40 per hour can be considered High (Severe) sleep Apnea.

The Advanced Apnea Management System will be used by hospitals, retirement centres, family members, who have the need to manage/monitor multiple people using their own Apnea Management System. This is like a multi-user management system, which can see vital signs, as configured, of multiple people, simultaneously.

All hardware/technologies required for the Input and Output devices currently exist. The Apnea Management System is the unique method that brings all the processes, functions and algorithms together to manage the devices within the Apnea, Snoring and SIDS arenas.

The aim is to provide a method of monitoring and management to reduce Apnea episodes and symptoms, assisting people to reduce the effects of Apnea and delay the need to use a CPAP machine in the first instance of their treatment plan.

### Interpretation

### Embodiments:

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly, it should be appreciated that in the above description of example embodiments of the invention, various features of the invention are sometimes grouped together in a single embodiment, figure, or description thereof for the purpose of streamlining the disclosure and aiding in the understanding of one or more of the various inventive aspects. This method of disclosure is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the Detailed Description of Specific Embodiments are hereby expressly incorporated into this Detailed Description of Specific Embodiments, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

### Different Instances of Objects

As used herein, unless otherwise specified the use of the ordinal adjectives "first", "second", "third", etc., to describe a common object, merely indicate that different instances of like objects are being referred to, and are not intended to imply that the objects so described must be in a given sequence, either temporally, spatially, in ranking, or in any other manner.

### Specific Details

In the description provided herein, numerous specific details are set forth. It is understood that embodiments of the invention may be practiced without these specific details. In other instances, well-known methods, structures and techniques have not been shown in detail in order not to obscure an understanding of this description. Terminology

In describing the preferred embodiment of the invention illustrated in the drawings, specific terminology will be resorted to for the sake of clarity. The invention is not intended to be limited to the specific terms so selected, and it is to be understood that each specific term includes all technical equivalents which operate in a similar manner to accomplish a similar technical purpose. Terms such as "forward", "rearward", "radially", "peripherally", "upwardly", "downwardly", and the like are used as words of convenience to provide reference points and are not to be construed as limiting terms.

### Comprising and Including

In the claims which follow and in the preceding description of the invention, except where the context requires otherwise due to express language or necessary implication, the word "comprise" or variations such as "comprises" or "comprising" are used in an inclusive sense, i.e. to specify the presence of the stated features but not to preclude the presence or addition of further features in various embodiments of the invention.

Any one of the terms: including or which includes or that includes as used herein is also an open term that also means including at least the elements/features that follow the term, but not excluding others. Thus, including is synonymous with and means comprising.

### Industrial Applicability

It is apparent from the above, that the arrangements described are applicable to the health industry and self-help health industry industries and in particular to the field of treatment, management and pre-management against disorders such as sleep apnea or snoring or sudden infant syndrome.

## Claims

1. A system for aiding early detection and management of a breathing related disorder in a patient comprising:
a. a user controller (11);
a. a plurality of sensors (20/30), comprising: an oximetry/O₂ level sensor and at least one additional sensor, wherein the sensors are connectable to the user controller and adapted for sensing and providing sensed data of at least one characteristic relevant to a breathing related disorder;
b. a predefined operative framework (15) in the user controller having connection for receiving the sensed data from the plurality of sensors;
c. a control means (53) for sending to the predefined operative framework a predefined effective operative range of the plurality of sensors;
d. a collaborator (120); and
e. at least one external stimulator (140) positionable relative to the patient and operatively connectable with the predefined operative framework (15) for receiving operative instruction and providing an effective external stimulation to the patient;
wherein:
i. the sensed data is assessed to determine whether a predefined trigger point has been sensed; and
ii. If a predefined trigger point has been sensed an identification of the single sensor trigger point is communicated to the collaborator (120); and
iii. The collaborator (120) undertakes a trigger point analysis by reviewing multiple sensors, whereby:
• if multiple single sensor trigger points from multiple sensors have been received within a predefined time period a trigger actuation is initiated to instigate external stimulation; or
• if multiple single sensor trigger points from multiple sensors have not been received within a predefined time period return to further sensing and further trigger analysis
wherein the recordal of a single sensor trigger point of one sensor does not initiate trigger actuation unless supported by a single sensor trigger point of another sensor.

2. The system according to claim 1, wherein:
i) the breathing related disorder is selected from the group consisting of:
a. sleep apnea;
b. snoring; and
a. SIDS (Sudden Infant Death Syndrome);
and
ii) the plurality of sensors are selected from sensors positionable at any one or more of the following external body sensor locations of:
a. Head (21);
b. Throat (29);
c. Nose (27);
d. Mouth (28);
e. Ear (22);
f. Wrist (24);
g. Finger (25);
h. foot/limb (26)
and
iii) the at least one additional sensor is selected from sensors adapted to sense at least one of the following:
a. pulse / heart rate (221);
b. temperature (223);
c. body movement (224);
d. breath (225).

3. The system according to claim 1 or claim 2, wherein the control means for providing to the predefined operative framework a predefined effective operative range of the at least one external stimulator (140); and wherein the predefined effective treatment range and control aids early detection and management of a breathing related disorder by an effective external stimulation.

4. The system according to claim 1, wherein the effective external stimulation by the at least one external stimulator:
i) effects an alteration to the patient's breathing; or
ii) indirectly effects an alteration to the patient' s breathing; or
iii) indirectly effects an alteration to the patient's breathing by notification to the patient; or
iv) substantially directly effects an alteration to the patient's breathing.

5. The system according to any one of claims 1 to 4, wherein the effective external stimulation by the at least one external stimulator (140) directly effecting an alteration to the patient's breathing is selected from one or more of:
a. an external stimulator locatable on or near the ear of the user providing acoustic output for stimulation to the patient;
b. an external stimulator locatable on or near the eye of the user providing light output for stimulation to the patient;
c. an external stimulator locatable under chin for vibration stimulation to the patient;
d. an external stimulator locatable on the head for external stimulation to the patient;
e. an external stimulator locatable on the finger for tactile stimulation to the patient;
f. an external stimulator using TENS (Transcutaneous Electrical Nerve Stimulation) or EMS (Electrical Muscle Stimulation), or both locatable to provide for stimulation to the patient; and
g. an external stimulator locatable on the foot for stimulation to the patient;wherein the external stimulators can operate in a mode that directly affects a patient to improve the breathing of the patient.

6. The system according to any one of claims 1 to 5, wherein the communication between sensors, user controller and at least one external stimulator is wireless.

7. The system according to claim 1, wherein the user controller (11) includes connection with one or more input selectable sensor modules providing the at least one sensor (20/30), and optionally wherein the one or more input selectable modules includes a selected sensor internal module part (99) for connection with the user controller and an external sensor module part (91) for location on an external body location and for providing sensed data to the selected sensor internal module part (99).

8. The system according to claim 7, wherein the external sensor module part is:
i) positionable to sense at one or more of the following external body sensor locations:
a. Head (21);
b. Throat (29);
c. Nose (27);
d. Mouth (28);
e. Ear (22);
f. Wrist (24);
g. Finger (25);
h. foot/limb (26);
and
ii) selected to sense according to at least one of the following modes of:
a. pulse / heart rate (221);
b. oximetry / O₂ levels (222);
c. temperature (223);
d. body movement (224);
e. breath (225).

9. The system according to claim 1, wherein the user controller (11) includes connection with one or more output selectable stimulator modules (191) providing the at least one external stimulator, and optionally wherein the one or more output selectable stimulator modules (181, 189, 191, 199) includes a selected stimulator internal module (199) part for connection with the user controller (11) and an external stimulator module part (191) for location on an external body location and for providing stimulation to the selected stimulator external module part.

10. The system according to claim 9, wherein the external stimulator module part is:
i) positionable to stimulate at one or more of the following external body sensor locations:
a. Head (21);
b. Throat (29);
c. Nose (27);
d. Mouth (28);
e. Ear (22);
f. Wrist (24);
g. Finger (25);
h. Foot (26);
and
ii) selected to sense according to at least one of the following modes of:
a. electrical pulse TENS / EMS;
b. sound/audio;
c. touch/physical;
d. vibration;
e. light.

11. A system for aiding management of a breathing related disorder comprising:
a. at least one sensor (20/30) for sensing at least one characteristic of a breathing related disorder;
b. an access port for upload of details of the at least one characteristic of a breathing related disorder from the sensor to an online computerised means;
c. a receiver port; for download from the online computerised means, which is adapted for receiving computerised instructions according to a predefined effective treatment range in managing control of a breathing related disorder;
d. at least one topical stimulator (171, 172, 173, 174, 175, 176) positionable on a user for providing an effective stimulation option to the user and including a receiver for receiving electronic control instructions;
e. at least one input device (140) for allowing a user to predefine at least one user particular stimulation output and provide for upload to the access port;
f. at least one display device (140) for allowing display of an external stimulation output to the user to provide a confirmed user defined management control of the breathing related disorder application;
g. at least one transmitter for transmitting each user particular input of the plurality of users to the access port of the online means for upload to the online computerised means; and
h. at least one receiver for transmitting for receiving by at least one of the at least one display device for allowing display of the respective confirmed user defined breathing related disorder application of each user;
wherein the plurality of user particular inputs defines the predefined effective treatment range and control of a breathing related disorder and the selection of an effective stimulation option defines the chosen effective option and is provided to the at least one display device.

12. The system according to claim 11, wherein the breathing related disorders include one or more of:
a. sleep apnea;
b. snoring; and
c. SIDS (Sudden Infant Death Syndrome).

13. The system according to claim 11, wherein the at least one input device provides a predefined operative framework identifying a plurality of stimulation options for selection by the user in their respective user particular input wherein a selection of an effective stimulation option from each category matching a user particular input of the same stimulation options in each category defines the chosen effective option.

14. The system according to claim 11, wherein the predefined operative framework identifying a plurality of stimulation options includes a plurality of categories or selection of at least one stimulation option in at least each of the plurality of categories by the user in their respective user particular input and optionally wherein there are at least 2 categories of stimulation options and wherein a selection of an effective stimulation option from each category matching a user particular input of the same stimulation options in each category defines the chosen effective option.

15. The system for aiding early detection and management of breathing related disorders according to claim 11, wherein the predefined operative framework identifying a plurality of stimulation options includes a plurality of categories or selection of at least one stimulation option in at least each of the plurality of categories by the user in their respective user particular input and wherein the categories of stimulation options include a plurality of:
a. stimulation option category;
b. adjusted stimulation option category; and
c. adjusting form of stimulation from a stimulation in the Stimulation option category.

16. The system for aiding early detection and management of breathing related disorders according to claim 11, wherein the predefined operative framework identifying a plurality of stimulation options includes a plurality of categories or selection of at least one stimulation option in at least each of the plurality of categories by the user in their respective user particular input and wherein there are at least 5 categories of stimulation options in the stimulation option category and wherein a selection of an effective stimulation option from each category matching a user particular input of the same stimulation options in each category defines the chosen effective option.

17. The system according to claim 11, wherein:
i) the adjusting form of stimulation from a stimulation in the stimulation option category is selected from:
a. acoustic;
b. physical; and
c. electrical pulse.
or
ii) the adjusting form of stimulation in the same stimulation option category is selected from:
a. variable selected frequency;
b. change of magnitude;
c. change of period; and
d. change of symmetry of stimulation.

18. The system according to claim 11, wherein the predefined operative framework identifying a plurality of stimulation options includes a plurality of categories includes a plurality of the stimulation option categories selected from:
a. foot;
b. wrist;
c. ear; and
d. head.

19. A method of automatically creating and running a freeform breathing related disorder using a computerised system including the steps of:
a. providing a predefined operative framework identifying a plurality of stimulation options for selection by the user in their respective user particular input wherein a selection of an effective stimulation option from each category matching a user particular input of the same stimulation options in each category defines the chosen effective option
b. receiving the user defined breathing related disorder application from a user over a digital communication system connected to an access port for upload to an online computerised means adapted for following computerised instructions according to a predefined effective treatment range and control of a breathing related disorder;
c. selecting an effective stimulation option according to the predefined operative framework;
d. comparing the plurality of effective stimulation options from each category to each of confirmed user defined breathing related disorder application wherein a selection of an effective stimulation option from each category matching a user particular input of the same stimulation options in each category defines the chosen effective option.

20. The method according to claim 19, wherein the predefined operative framework identifying a plurality of stimulation options includes a plurality of categories or selection of at least one stimulation option in at least each of the plurality of categories by the user in their respective user particular input and wherein:
i) there are at least 5 categories of stimulation options and wherein a selection of an effective stimulation option from each category matching a user particular input of the same stimulation options in each category defines the chosen effective option;
or
ii) there are at least 8 categories of stimulation options and wherein a selection of a effective stimulation option from each category matching a user particular input of the same stimulation options in each category defines the chosen effective option;
or.
iii) the categories of stimulation options include a plurality of:
a. stimulation option category;
b. adjusted stimulation option category; and
c. adjusting form of stimulation from a stimulation in the Stimulation option category.

21. The method according to claim 19, further including:
i) the step of providing a plurality of categories for defining at least one selection must be made in each category; or
ii) providing a plurality of stimulation options each allocated to one only of the plurality of categories;

22. The method according to claim 19, wherein the adjusting form of stimulation from a stimulation in the stimulation option category is selected from:
a. variable selected frequency;
b. magnitude;
c. period; and
d. symmetry of stimulation.

23. The method according to claim 19, wherein the predefined operative framework identifying a plurality of stimulation options includes a plurality of categories includes a plurality of the stimulation option categories selected from:
a. foot;
b. wrist;
c. ear; and
d. head.

## Patentansprüche

1. System zum Unterstützen der Früherkennung und Verwaltung einer Atmungsstörung bei einem Patienten, umfassend:
a. eine Benutzersteuerung (11);
a. mehrere Sensoren (20/30), umfassend: einen Oximetrie-/O₂-Gehaltssensor und zumindest einen zusätzlichen Sensor, wobei die Sensoren mit der Benutzersteuerung verbunden sind und zum Erfassen und Zuführen von erfassten Daten von zumindest einem Kennzeichen, das für eine Atmungsstörung relevant ist, geeignet sind;
b. einen vordefinierten operativen Rahmen (15) in der Benutzersteuerung mit Verbindung zum Empfangen der erfassten Daten von den mehreren Sensoren;
c. ein Steuermittel (53) zum Senden einer vordefinierten effektiven operativen Reichweite der mehreren Sensoren an den vordefinierten operativen Rahmen;
d. einen Kollaborator (120); und
e. zumindest einen externen Stimulator (140), der bezüglich des Patienten positionierbar ist und betriebsfähig mit dem vordefinierten operativen Rahmen (15) zum Empfangen von operativer Anweisung und Zuführen einer effektiven externen Stimulation zum Patienten verbindbar ist;
wobei:
i. die erfassten Daten beurteilt werden, um zu bestimmen, ob ein vordefinierter Auslösepunkt erfasst wurde; und
ii. wenn ein Auslösepunkt erfasst wurde, dem Kollaborator (120) eine Identifikation des einzelnen Sensorauslösepunkts übermittelt wird; und
iii. der Kollaborator (120) eine Auslösepunktanalyse durch Überprüfen von mehrfachen Sensoren unternimmt, wodurch:
- wenn mehrfache einzelne Sensorauslösepunkte von mehrfachen Sensoren innerhalb einer vordefinierten Zeitperiode empfangen wurden, eine Auslösebetätigung zum Anregen von externer Stimulation eingeleitet wird; oder
- wenn mehrfache einzelne Sensorauslösepunkte von mehrfachen Sensoren nicht innerhalb einer vordefinierten Zeitperiode empfangen wurden, zu weiterem Erfassen und weiterer Auslöseanalyse zurückgekehrt wird,
wobei die Erfassung eines einzelnen Sensorauslösepunkts von einem Sensor keine Auslösebetätigung einleitet, solange sie nicht durch einen einzelnen Sensorauslösepunkt eines anderen Sensors bestätigt wird.

2. System nach Anspruch 1, wobei:
i) die Atmungsstörung aus der Gruppe ausgewählt ist, die aus Folgendem besteht:
a. Schlafapnoe;
b. Schnarchen; und
a. SIDS (Sudden Infant Death Syndrome; plötzlicher Kindstod);
und
ii) die mehreren Sensoren aus Sensoren ausgewählt sind, die an einer oder mehr der folgenden externen Körpersensorstellen positionierbar sind:
a. Kopf (21);
b. Hals (29);
c. Nase (27);
d. Mund (28);
e. Ohr (22);
f. Handgelenk (24);
g. Finger (25);
h. Fuß/Körperglied (26),
und
iii) der zumindest eine zusätzliche Sensor aus Sensoren ausgewählt ist, die zum Erfassen von zumindest einem des Folgenden geeignet sind:
a. Puls-/Herzschlag (221);
b. Temperatur (223);
c. Körperbewegung (224);
d. Atem (225).

3. System nach Anspruch 1 oder 2, wobei das Steuermittel dem Vorsehen einer vordefinierten effektiven operativen Reichweite des zumindest einen Stimulators (140) für den vordefinierten operativen Rahmen dient; und wobei der vordefinierte effektive Behandlungsrahmen und die Steuerung bei der Früherkennung und Verwaltung einer Atemstörung durch eine effektive externe Stimulation unterstützen.

4. System nach Anspruch 1 oder 2, wobei die effektive externe Stimulation durch den zumindest einen externen Stimulator:
i) eine Änderung an der Atmung des Patienten ausführt; oder
ii) indirekt eine Änderung an der Atmung des Patienten ausführt; oder
iii) indirekt eine Änderung an der Atmung des Patienten durch Benachrichtigung des Patienten ausführt; oder
iv) im Wesentlichen direkt eine Änderung an der Atmung des Patienten ausführt.

5. System nach einem der Ansprüche 1 bis 4, wobei die effektive externe Stimulation durch den zumindest einen Stimulator (140), die eine Änderung an der Atmung des Patienten direkt ausführt, aus einem oder mehr des Folgenden ausgewählt ist:
a. einem externen, am oder nahe am Ohr des Benutzers befindlichen Stimulator, der akustische Ausgabe zur Stimulierung des Patienten zuführt;
b. einem externen, am oder nahe am Auge des Benutzers befindlichen Stimulator, der Lichtausgabe zur Stimulierung des Patienten zuführt;
c. einem externen, unter dem Kinn befindlichen Stimulator zur Vibrationsstimulation des Patienten;
d. einem externen, am Kopf befindlichen Stimulator zur Berührungsstimulation des Patienten;
e. einem externen, am Finger befindlichen Stimulator zur Berührungsstimulation des Patienten;
f. einem externen Stimulator, der TENS (Transcutaneous Electrical Nerve Stimulation; transkutane elektrische Nervenstimulation) oder EMS (Electrical Muscle Stimulation; elektrische Muskelstimulation) oder beides nutzt, zum Zuführen von Stimulation zum Patienten; und
g. einem externen, am Fuß befindlichen Stimulator zur Stimulation des Patienten; wobei die externen Stimulatoren in einem Modus arbeiten können, der sich direkt auf einen Patienten auswirkt, um die Atmung des Patienten zu verbessern.

6. System nach einem der Ansprüche 1 bis 5, wobei die Kommunikation zwischen Sensoren, Benutzersteuerung und zumindest einem externen Stimulator drahtlos ist.

7. System nach Anspruch 1, wobei die Benutzersteuerung (11) Verbindung mit einem oder mehr eingangswählbaren Sensormodulen enthält, die den zumindest einen Sensor (20/30) vorsehen, und wobei die ein oder mehr eingangswählbaren Module optional ein ausgewähltes sensorinternes Modulteil (99) zur Verbindung mit der Benutzersteuerung und ein externes Sensormodulteil (91) zur Anordnung an einer externen Körperstelle und zum Zuführen von erfassten Daten zum ausgewählten sensorinternen Modulteil (99) enthalten.

8. System nach Anspruch 7, wobei das externe Sensormodulteil:
i) zum Erfassen an einer oder mehr der folgenden externen Körpersensorstellen positionierbar ist:
a. Kopf (21);
b. Hals (29);
c. Nase (27);
d. Mund (28);
e. Ohr (22);
f. Handgelenk (24);
g. Finger (25);
h. Fuß/Körperglied (26),
und
ii) zum Erfassen gemäß zumindest einem der folgenden Modi ausgewählt ist:
a. Puls-/Herzschlag (221);
b. Oximetrie/O2-Gehalt (222);
c. Temperatur (223);
d. Körperbewegung (224);
e. Atem (225).

9. System nach Anspruch 1, wobei die Benutzersteuerung (11) Verbindung mit einem oder mehr ausgangswählbaren Stimulatormodulen (191) enthält, die den zumindest einen externen Stimulator vorsehen, und wobei die ein oder mehr ausgangswählbaren Stimulatormodule (181, 189, 191, 199) optional ein ausgewähltes stimulatorinternes Modulteil (199) zur Verbindung mit der Benutzersteuerung (11) und ein externes Stimulatormodulteil (191) zur Anordnung an einer externen Körperstelle und zum Zuführen von Stimulation zum ausgewählten stimulatorexternen Modulteil enthalten.

10. System nach Anspruch 9, wobei das externe Stimulatormodulteil:
i) zum Stimulieren an einem oder mehr der folgenden externen Körpersensorstellen positionierbar ist:
a. Kopf (21);
b. Hals (29);
c. Nase (27);
d. Mund (28);
e. Ohr (22);
f. Handgelenk (24);
g. Finger (25);
h. Fuß/Körperglied (26),
und
ii) zum Erfassen gemäß zumindest einem der folgenden Modi ausgewählt ist:
a. elektrische Impulse TENS/EMS;
b. Ton/Audio;
c. Berührung/physisch;
d. Vibration;
e. Licht.

11. System zum Unterstützen der Verwaltung einer Atmungsstörung, umfassend:
a. zumindest einen Sensor (20/30) zum Erfassen von zumindest einem Kennzeichen einer Atmungsstörung;
b. einen Zugangsanschluss zum Hochladen von Details des zumindest einen Kennzeichens einer Atmungssteuerung vom Sensor auf ein computergestütztes Online-Mittel;
c. einen Empfängeranschluss zum Herunterladen vom computergestützten Onlinemittel, der zum Empfangen von computergestützten Anweisungen gemäß einer vordefinierten effektiven Behandlungsreichweite bei der Verwaltungssteuerung einer Atmungsstörung geeignet ist;
d. zumindest einen topischen Stimulator (171, 172, 173, 174, 175, 176), der an einem Benutzer zum Zuführen einer effektiven Stimulationsoption positionierbar ist und einen Empfänger zum Empfangen von elektronischen Steueranweisungen enthält;
e. zumindest ein Eingabegerät (140) zum Ermöglichen, dass der Benutzer zumindest eine benutzerspezifische Stimulationsausgabe vordefiniert und zum Hochladen zum Zugangsanschluss vorsieht;
f. zumindest ein Anzeigegerät (140) zum Ermöglichen des Anzeigens einer externen Stimulationsausgabe für den Benutzer zum Vorsehen einer bestätigten benutzerdefinierten Verwaltungssteuerung der Atmungsstörungsanwendung;
g. zumindest einen Sender zum Senden jeder benutzerspezifischen Eingabe der mehreren Benutzer an den Zugangsanschluss des Online-Mittels zum Hochladen auf das computergestützte OnlineMittel; und
h. zumindest einen Empfänger zum Empfangen durch zumindest eines des zumindest einen Anzeigegeräts zum Ermöglichen der Anzeige der jeweiligen bestätigten benutzerdefinierten Atemstörungsanwendung jeden Benutzers;
wobei die mehreren benutzerspezifischen Eingaben die vordefinierte effektive Behandlungsreichweite und Steuerung einer Atmungsstörung definieren und die Auswahl einer effektiven Stimulationsoption die gewählte effektive Option definiert und dem zumindest einen Anzeigegerät zugeführt wird.

12. System nach Anspruch 11, wobei die Atmungsstörungen eines oder mehr von Folgendem beinhalten:
a. Schlafapnoe;
b. Schnarchen; und
c. SIDS (Sudden Infant Death Syndrome; plötzlicher Kindstod).

13. System nach Anspruch 11, wobei das zumindest eine Eingabegerät einen vordefinierten operativen Rahmen vorsieht, der mehrere Stimulationsoptionen zur Auswahl durch die Benutzer in ihrer jeweiligen benutzerspezifischen Eingabe identifiziert, wobei eine Auswahl einer effektiven Stimulationsoption aus jeder Kategorie, die mit einer benutzerspezifischen Eingabe derselben Stimulationsoptionen in jeder Kategorie übereinstimmt, die gewählte effektive Option definiert.

14. System nach Anspruch 11, wobei der vordefinierte operative Rahmen, der mehrere Stimulationsoptionen identifiziert, mehrere Kategorien oder die Auswahl von zumindest einer Stimulationsoption in zumindest jeder der mehreren Kategorien durch die Benutzer in ihrer jeweiligen benutzerspezifischen Eingabe enthält, und wobei optional zumindest 2 Kategorien von Stimulationsoptionen vorliegen, und wobei eine Auswahl einer effektiven Stimulationsoption aus jeder Kategorie, die mit einer benutzerspezifischen Eingabe derselben Stimulationsoptionen in jeder Kategorie übereinstimmt, die gewählte effektive Option definiert.

15. System zum Unterstützen der Früherkennung und Verwaltung von Atmungsstörungen nach Anspruch 11, wobei der vordefinierte operative Rahmen, der mehrere Stimulationsoptionen identifiziert, mehrere Kategorien oder die Auswahl von zumindest einer Stimulationsoption in zumindest jeder der mehreren Kategorien durch die Benutzer in ihrer jeweiligen benutzerspezifischen Eingabe enthält, und wobei die Kategorien von Stimulationsoptionen eine Vielzahl von Folgenden enthalten:
a. Stimulationsoptionskategorie;
b. angepasste Stimulationskategorie; und
c. Anpassen der Stimulationsform von einer Stimulation in der Stimulationsoptionskategorie.

16. System zum Unterstützen der Früherkennung und Verwaltung von Atmungsstörungen nach Anspruch 11, wobei der vordefinierte operative Rahmen, der mehrere Stimulationsoptionen identifiziert, mehrere Kategorien oder die Auswahl von zumindest einer Stimulationsoption in zumindest jeder der mehreren Kategorien durch die Benutzer in ihrer jeweiligen benutzerspezifischen Eingabe enthält, und wobei zumindest 5 Kategorien von Stimulationsoptionen in der Stimulationsoptionskategorie vorliegen, und wobei eine Auswahl einer effektiven Stimulationsoption aus jeder Kategorie, die mit einer benutzerspezifischen Eingabe derselben Stimulationsoptionen in jeder Kategorie übereinstimmt, die gewählte effektive Option definiert.

17. System nach Anspruch 11, wobei:
i) das Anpassen der Stimulationsform von einer Stimulation in der Stimulationsoptionskategorie ausgewählt ist aus:
a. akustisch;
b. physisch; und
c. elektrischer Impuls;
oder
ii) das Anpassen der Stimulationsform von einer Stimulation in der Stimulationsoptionskategorie ausgewählt ist aus:
a. variabel ausgewählte Frequenz;
b. Änderung der Größe;
c. Änderung der Periode; und
d. Änderung der Stimulationssymmetrie.

18. System nach Anspruch 11, wobei der vordefinierte operative Rahmen, der mehrere Stimulationsoptionen identifiziert, mehrere Kategorien enthält, und mehrere Stimulationsoptionskategorien enthält, die ausgewählt sind aus:
a. Fuß;
b. Handgelenk;
c. Ohr; und
d. Kopf.

19. Verfahren zum automatischen Erzeugen und Ausführen einer Freiformatmungsstörung unter Benutzung eines computergestützten Systems, die folgenden Schritte enthaltend:
a. Vorsehen eines vordefinierten operativen Rahmens, der mehrere Stimulationsoptionen zur Auswahl durch die Benutzer in ihrer jeweiligen benutzerspezifischen Eingabe identifiziert, wobei eine Auswahl einer effektiven Stimulationsoption aus jeder Kategorie, die mit einer benutzerspezifischen Eingabe derselben Stimulationsoptionen in jeder Kategorie übereinstimmt, die gewählte effektive Option definiert;
b. Empfangen der benutzerdefinierten Atemstörungsanwendung von einem Benutzer über ein digitales Kommunikationssystem, das mit einem Zugangsanschluss zum Hochladen auf ein computergestütztes Online-Mittel verbunden ist, welches zum Befolgen von computergestützten Anweisungen gemäß einer vordefinierten effektiven Behandlungsreichweite und Steuerung einer Atmungsstörung geeignet ist;
c. Auswählen einer effektiven Stimulationsoption gemäß dem vordefinierten operativen Rahmen;
d. Vergleichen der mehreren effektiven Stimulationsoptionen aus jeder Kategorie mit jeder bestätigten benutzerdefinierten Atmungsstörungsanwendung, wobei eine Auswahl einer effektiven Stimulationsoption aus jeder Kategorie, die mit einer benutzerspezifischen Eingabe derselben Stimulationsoptionen in jeder Kategorie übereinstimmt, die gewählte effektive Option definiert.

20. Verfahren nach Anspruch 19, wobei der vordefinierte operative Rahmen, der mehrere Stimulationsoptionen identifiziert, mehrere Kategorien oder die Auswahl von zumindest einer Stimulationsoption in zumindest jeder der mehreren Kategorien durch die Benutzer in ihrer jeweiligen benutzerspezifischen Eingabe enthält, und wobei:
i) zumindest 5 Kategorien von Stimulationsoptionen vorliegen, und wobei eine Auswahl einer effektiven Stimulationsoption aus jeder Kategorie, die mit einer benutzerspezifischen Eingabe derselben Stimulationsoptionen in jeder Kategorie übereinstimmt, die gewählte effektive Option definiert;
oder
ii) zumindest 8 Kategorien von Stimulationsoptionen vorliegen, und wobei eine Auswahl einer effektiven Stimulationsoption aus jeder Kategorie, die mit einer benutzerspezifischen Eingabe derselben Stimulationsoptionen in jeder Kategorie übereinstimmt, die gewählte effektive Option definiert;
oder
iii) die Kategorien von Stimulationsoptionen eine Vielzahl von Folgenden enthalten:
a. Stimulationsoptionskategorie;
b. angepasste Stimulationskategorie; und
c. Anpassen der Stimulationsform von einer Stimulation in der Stimulationsoptionskategorie.

21. Verfahren nach Anspruch 19, ferner enthaltend:
i) der Schritt des Vorsehens von mehreren Kategorien zum Definieren von zumindest einer Auswahl muss in jeder Kategorie ausgeführt werden; oder
ii) Vorsehen von mehreren Stimulationsoptionen, die jede nur einer der mehreren Kategorien zugeordnet sind.

22. Verfahren nach Anspruch 19, wobei das Anpassen der Stimulationsform von einer Stimulation in der Stimulationsoptionskategorie ausgewählt ist aus:
a. variabel ausgewählte Frequenz;
b. Größe;
c. Periode; und
d. Stimulationssymmetrie.

23. Verfahren nach Anspruch 19, wobei der vordefinierte operative Rahmen, der mehrere Stimulationsoptionen identifiziert, mehrere Kategorien enthält, und mehrere Stimulationsoptionskategorien enthält, die ausgewählt sind aus:
a. Fuß;
b. Handgelenk;
c. Ohr; und
d. Kopf.

## Revendications

1. Système pour faciliter une détection et une prise en charge précoces d'un trouble lié à la respiration chez un patient comprenant:
a. un dispositif de commande utilisateur (11);
b. une pluralité de capteurs (20/30) comprenant: un capteur d'oxymétrie/de niveau de O₂ et au moins un capteur supplémentaire, dans lequel les capteurs peuvent être connectés au dispositif de commande utilisateur et adaptés pour détecter et fournir des données détectées d'au moins une caractéristique pertinente pour un trouble lié à la respiration;
c. un cadre opératoire prédéfini (15) dans le dispositif de commande utilisateur ayant une connexion pour recevoir les données détectées de la pluralité de capteurs;
d. un moyen de commande (53) pour envoyer au cadre opératoire prédéfini une plage opérationnelle efficace prédéfinie de la pluralité de capteurs;
e. un collaborateur (120); et
f. au moins un stimulateur externe (140) pouvant être positionné par rapport au patient et pouvant être connecté de manière fonctionnelle au cadre opératoire prédéfini (15) pour recevoir des instructions opératoires et fournir une stimulation externe efficace au patient;
dans lequel:
i. les données détectées sont évaluées pour déterminer si un point de déclenchement prédéfini a été détecté; et
ii. si un point de déclenchement prédéfini a été détecté, une identification du point de déclenchement de capteur unique est communiquée au collaborateur (120); et
iii.le collaborateur (120) entreprend une analyse de point de déclenchement en examinant de multiples capteurs, de sorte que:
• si de multiples points de déclenchement de capteur unique provenant de multiples capteurs ont été reçus dans une période de temps prédéfinie, un actionnement de déclenchement est initié pour provoquer une stimulation externe; ou
• si de multiples points de déclenchement de capteur unique provenant de multiples capteurs n'ont pas été reçus dans une période de temps prédéfinie, revenir à une détection supplémentaire et une analyse de déclenchement supplémentaire
où l'enregistrement d'un point de déclenchement de capteur unique d'un détecteur n'initie pas un actionnement de déclenchement sauf s'il est soutenu par un point de déclenchement de capteur unique d'un autre capteur.

2. Système selon la revendication 1, dans lequel:
i) le trouble lié à la respiration est sélectionné dans le groupe consistant en:
a. l'apnée du sommeil;
b. le ronflement; et
a. le SMSN (syndrome de mort subite du nourrisson); et
ii) la pluralité de capteurs sont sélectionnés parmi des capteurs positionnables à l'un quelconque ou plusieurs des emplacements de capteur corporel externe suivants de:
a. la tête (21);
b. la gorge (29);
c. le nez (27);
d. la bouche (28);
e. l'oreille (22);
f. le poignet (24);
g. le doigt (25);
h. le pied/membre (26)
et
iii) le au moins un capteur supplémentaire est sélectionné parmi les capteurs adaptés pour détecter au au moins l'un des suivants:
a. le pouls/rythme cardiaque (221);
b. la température (223);
c. le mouvement corporel (224);
d. la respiration (225).

3. Système selon la revendication 1 ou la revendication 2, dans lequel les moyens de commande pour fournir au cadre opératoire prédéfini une plage opératoire efficace prédéfinie du au moins un stimulateur externe (140); et dans lequel la plage de traitement efficace prédéfinie et la commande facilitent la détection et la prise en charge précoces d'un trouble lié à la respiration par une stimulation externe efficace.

4. Système selon la revendication 1, dans lequel la stimulation externe efficace par le au moins un stimulateur externe:
i) provoque une altération de la respiration du patient; ou
ii) provoque indirectement une altération de la respiration du patient; ou
iii) provoque indirectement une altération de la respiration du patient par notification au patient,
ou
iv) provoque sensiblement directement une altération de la respiration du patient.

5. Système selon l'une quelconque des revendications 1 à 4, dans lequel la stimulation externe efficace par le au moins un stimulateur externe (140) provoquant directement une altération de la respiration du patient est sélectionnée parmi un ou plusieurs de:
a. un stimulateur externe localisable sur ou près de l'oreille de l'utilisateur fournissant une sortie acoustique pour la stimulation au patient;
b. un stimulateur externe localisable sur ou près de l'œil de l'utilisateur fournissant une sortie lumineuse pour la stimulation au patient;
c. un stimulateur externe localisable sous le menton pour la stimulation par vibration au patient;
d. un stimulateur externe localisable sur la tête pour une stimulation externe au patient;
e. un stimulateur externe localisable sur le doigt pour une stimulation tactile au patient;
f. un stimulateur externe utilisant la TENS (stimulation nerveuse électrique transcutanée) ou l'EMS (stimulation musculaire électrique), ou les deux localisables pour fournir une stimulation au patient; et
g. un stimulateur externe localisable sur le pied pour la stimulation au patient; dans lequel les stimulateurs externes peuvent fonctionner dans un mode qui affecte directement un patient pour améliorer la respiration du patient.

6. Système selon l'une quelconque des revendications 1 à 5, dans lequel la communication entre les capteurs, dispositif de commande utilisateur et au moins un stimulateur externe est sans fil.

7. Système selon la revendication 1, dans lequel le dispositif de commande utilisateur (11) inclut une connexion avec un ou plusieurs modules de capteur sélectionnables en entrée fournissant le au moins un capteur (20/30), et éventuellement dans lequel les ou plusieurs modules sélectionnables en entrée incluent une partie de module interne de capteur sélectionné (99) pour une connexion avec le dispositif de commande utilisateur et une partie de module de capteur externe (91) pour une localisation sur un emplacement corporel externe et pour fournir des données détectées à la partie de module interne de capteur sélectionné (99).

8. Système selon la revendication 7, dans lequel la partie de module de capteur externe est:
i) positionnable pour détecter à un ou plusieurs des emplacements de capteur corporel externe suivants:
a. la tête (21);
b. la gorge (29);
c. le nez (27);
d. la bouche (28);
e. l'oreille (22);
f. le poignet (24);
g. le doigt (25);
h. le pied/membre (26);
et
ii) sélectionnée pour détecter selon au moins l'un des modes suivants de:
a. le pouls/rythme cardiaque (221);
b. l'oxymétrie/niveaux de O₂ (222);
c. la température (223);
d. le mouvement corporel (224);
e. la respiration (225).

9. Système selon la revendication 1, dans lequel le dispositif de commande utilisateur (11) inclut une connexion avec un ou plusieurs modules de stimulation sélectionnables en sortie (191) fournissant le au moins un stimulateur externe, et éventuellement dans lequel les un ou plusieurs modules de stimulation sélectionnables en sortie (181, 189, 191, 199) incluent une partie de module interne de stimulateur sélectionné (199) pour une connexion avec le dispositif de commande utilisateur (11) et une partie de module de stimulateur externe (191) pour une localisation sur un emplacement corporel externe et pour fournir une stimulation à la partie de module externe de stimulateur sélectionné.

10. Système selon la revendication 9, dans lequel la partie de module de stimulateur externe est:
i) positionnable pour stimuler à un ou plusieurs des emplacements de capteur corporel externe suivants:
a. la tête (21);
b. la gorge (29);
c. le nez (27);
d. la bouche (28);
e. l'oreille (22);
f. le poignet (24);
g. le doigt (25);
h. le pied (26);
et
ii) sélectionnée pour détecter selon au moins l'un des modes suivants de:
a. impulsion électrique TENS/EMS;
b. son/audio;
c. tactile/physique;
d. vibration;
e. lumière.

11. Système pour faciliter la prise en charge d'un trouble lié à la respiration comprenant:
a. au moins un capteur (20/30) pour détecter au moins une caractéristique d'un trouble lié à la respiration;
b. un port d'accès pour le chargement de détails de la au moins une caractéristique d'un trouble lié à la respiration du capteur à un moyen informatisé en ligne;
c. un port récepteur; pour décharger à partir du moyen informatisé en ligne, qui est adapté pour recevoir des instructions informatisées selon une plage de traitement efficace prédéfinie dans la commande de prise en charge d'un trouble lié à la respiration;
d. au moins un stimulateur topique (171, 172, 173, 174, 175, 176) positionnable sur un utilisateur pour fournir une option de stimulation efficace à l'utilisateur et incluant un récepteur pour recevoir des instructions de commande électroniques;
e. au moins un dispositif d'entrée (140) pour permettre à un utilisateur de prédéfinir au moins une sortie de stimulation particulière à l'utilisateur et assurer le chargement vers le port d'accès;
f. au moins un dispositif d'affichage (140) pour permettre l'affichage d'une sortie de stimulation externe à l'utilisateur pour fournir une commande de prise en charge définie par l'utilisateur confirmée de l'application de trouble lié à la respiration;
g. au moins un émetteur pour transmettre chaque entrée particulière à l'utilisateur de la pluralité d'utilisateurs au port d'accès du moyen en ligne pour le chargement vers le moyen informatisé en ligne; et
h. au moins un récepteur pour transmettre pour la réception par au moins l'un du au moins un dispositif d'affichage pour permettre l'affichage de l'application de trouble lié à la respiration définie par l'utilisateur confirmée respective de chaque utilisateur;
dans lequel la pluralité d'entrées particulières à utilisateur définit la plage de traitement efficace prédéfinie et la commande d'un trouble lié à la respiration et la sélection d'une option de stimulation efficace définit l'option efficace choisie et est fournie au au moins un dispositif d'affichage.

12. Système selon la revendication 11, dans lequel les troubles liés à la respiration incluent un ou plusieurs de:
a. l'apnée du sommeil;
b. le ronflement; et
c. le SMSN (syndrome de mort subite du nourrisson).

13. Système selon la revendication 11, dans lequel le au moins un dispositif d'entrée fournit un cadre opératoire prédéfini identifiant une pluralité d'options de stimulation pour la sélection par l'utilisateur dans leur entrée particulière à l'utilisateur respective dans lequel une sélection d'une option de stimulation efficace dans chaque catégorie coïncidant avec une entrée particulière à l'utilisateur des mêmes options de stimulation dans chaque catégorie définit l'option efficace choisie.

14. Système selon la revendication 11, dans lequel le cadre opératoire prédéfini identifiant une pluralité d'options de stimulation inclut une pluralité de catégories ou la sélection d'au moins une option de stimulation dans au moins chacune de la pluralité de catégories par l'utilisateur dans leur entrée particulière à l'utilisateur respective et éventuellement dans lequel il y a au moins 2 catégories d'options de stimulation et dans lequel une sélection d'une option de stimulation efficace dans chaque catégorie coïncidant avec une entrée particulière à l'utilisateur des mêmes options de stimulation dans chaque catégorie définit l'option efficace choisie.

15. Système pour faciliter la détection et à la prise en charge précoces de troubles liés à la respiration selon la revendication 11, dans lequel le cadre opératoire prédéfini identifiant une pluralité d'options de stimulation inclut une pluralité de catégories ou la sélection d'au moins une option de stimulation dans au moins chacune de la pluralité de catégories par l'utilisateur dans leur entrée particulère à utilisateur respective et dans lequel les catégories d'options de stimulation incluent une pluralité de:
a. catégorie d'options de stimulation;
b. catégorie d'options de stimulation ajustée; et
c. forme d'ajustement de la stimulation à partir d'une stimulation dans la catégorie d'options de stimulation.

16. Système pour faciliter la détection et à la prise en charge précoces de troubles liés à la respiration selon la revendication 11, dans lequel le cadre opératoire prédéfini identifiant une pluralité d'options de stimulation inclut une pluralité de catégories ou la sélection d'au moins une option de stimulation dans au moins chacune de la pluralité de catégories par l'utilisateur dans leur entrée particulière à utilisateur respective et dans lequel il y a au moins 5 catégories d'options de stimulation dans la catégorie d'options de stimulation et dans lequel une sélection d'une option de stimulation efficace dans chaque catégorie coïncidant avec une entrée particulière à l'utilisateur des mêmes options de stimulation dans chaque catégorie définit l'option effective choisie.

17. Système selon la revendication 11, dans lequel:
i) la forme d'ajustement de la stimulation à partir d'une stimulation dans la catégorie d'options de stimulation est sélectionnée parmi:
a. acoustique;
b. physique; et
c. impulsion électrique.
ou
ii) la forme d'ajustement de la stimulation dans la même catégorie d'options de stimulation est sélectionnée parmi:
a. une fréquence sélectionnée variable;
b. un changement d'amplitude;
c. un changement de période; et
d. un changement de symétrie de stimulation.

18. Système selon la revendication 11, dans lequel le cadre opératoire prédéfini identifiant une pluralité d'options de stimulation inclut une pluralité de catégories inclut une pluralité des catégories d'options de stimulation sélectionnées parmi:
a. le pied;
b. le poignet;
c. l'oreille; et
d. la tête.

19. Procédé de création et d'exécution automatiques d'un trouble lié à la respiration de forme libre à l'aide d'un système informatisé incluant les étapes de:
a. fournir un cadre opératoire prédéfini identifiant une pluralité d'options de stimulation pour la sélection par l'utilisateur dans leur entrée particulière à l'utilisateur respective, dans lequel une sélection d'une option de stimulation efficace dans chaque catégorie coïncidant avec une entrée particulière à l'utilisateur des mêmes options de stimulation dans chaque catégorie définit l'option efficace choisie
b. recevoir l'application de trouble lié à la respiration définie par l'utilisateur depuis un utilisateur sur un système de communication numérique connecté à un port d'accès pour charger vers un moyen informatisé en ligne adapté pour suivre des instructions informatisées selon une plage de traitement efficace prédéfinie et commander un trouble lié à la respiration;
c. sélectionner une option de stimulation efficace selon le cadre opératoire prédéfini;
d. comparer la pluralité d'options de stimulation efficace dans chaque catégorie à chacune des applications de troubles liés à la respiration définies par l'utilisateur, dans lequel une sélection d'une option de stimulation efficace dans chaque catégorie coïncidant avec une entrée particulière à l'utilisateur des mêmes options de stimulation dans chaque catégorie définit l'option efficace choisie.

20. Procédé selon la revendication 19, dans lequel le cadre opératoire prédéfini identifiant une pluralité d'options de stimulation inclut une pluralité de catégories ou la sélection d'au moins une option de stimulation dans au moins chacune de la pluralité de catégories par l'utilisateur dans leur entrée particulière à l'utilisateur respective et dans lequel:
i) il y a au moins 5 catégories d'options de stimulation et dans lequel une sélection d'une option de stimulation efficace dans chaque catégorie coïncidant avec une entrée particulière à l'utilisateur des mêmes options de stimulation dans chaque catégorie définit l'option efficace choisie;
ou
ii) il y a au moins 8 catégories d'options de stimulation et dans lequel une sélection d'une option de stimulation efficace dans chaque catégorie coïncidant avec une entrée particulière à l'utilisateur des mêmes options de stimulation dans chaque catégorie définit l'option efficace choisie; ou.
iii) les catégories d'options de stimulation incluent une pluralité de:
a. catégorie d'options de stimulation;
b. catégorie d'options de stimulation ajustée; et
c. forme d'ajustement de la stimulation à partir d'une stimulation dans la catégorie d'options de stimulation.

21. Procédé selon la revendication 19, incluant en outre:
i) l'étape de fourniture d'une pluralité de catégories pour définir au moins une sélection doit être effectuée dans chaque catégorie; ou
ii) fourniture d'une pluralité d'options de stimulation attribuées chacune à une seule de la pluralité de catégories;

22. Procédé selon la revendication 19, dans lequel la forme d'ajustement de la stimulation à partir d'une stimulation dans la catégorie d'options de stimulation est sélectionnée parmi:
a. fréquence sélectionnée variable;
b. amplitude;
c. période; et
d. symétrie de stimulation.

23. Procédé selon la revendication 19, dans lequel le cadre opératoire prédéfini identifiant une pluralité d'options de stimulation inclut une pluralité de catégories inclut une pluralité des catégories d'options de stimulation sélectionnées parmi:
a. le pied;
b. le poignet;
c. l'oreille; et
d. la tête.
